# EUROPÄISCHE PATENTSCHRIFT

(11) **EP 1 629 842 B1**
(45) Veröffentlichungstag und Bekanntmachung des Hinweises auf die Patenterteilung: **14.04.2010**
(21) Anmeldenummer: 05024999.4
(22) Anmeldetag: 10.06.2002
(51) Int. Cl.: A61K 31/426, A61K 31/427, A61K 9/06, A61P 31/12

(54) **Topische Anwendung von Thiazolylamiden**
Topical application of thiazolyl amides
Utilisation topique d'amides de thiazolyle

(30) Priorität: 22.06.2001 DE 10129714
(43) Veröffentlichungstag der Anmeldung: 01.03.2006
(62) Teilanmeldung aus: 02743172.5
(73) Patentinhaber: AiCuris GmbH & Co. KG, 42117 Wuppertal (DE)
(72) Erfinder: Betz, Ulrich, Dr., 64354 Reinheim (DE); Laich, Tobias, Dr., 51069 Köln (DE); Bender, Wolfgang, Dr., 42113 Wuppertal (DE); Fischer, Rüdiger, Dr., 50259 Pulheim (DE); Hendrix, Martin, Dr., 51519 Odenthal (DE); Kleymann, Gerald, Dr., 32107 Bad Salzuflen (DE)
(74) Vertreter: Findeisen, Marco

(56) Entgegenhaltungen:
- WO-A-00/53591
- WO-A-01/47904
- WO-A-01/96874
- KLEYMANN, GERALD ET AL: "New helicase-primase inhibitors as drug candidates for the treatment of herpes simplex disease" NATURE MEDICINE (NEW YORK, NY, UNITED STATES) (2002), 8(4), 392-398, April 2002 (2002-04), XP001105918

## Beschreibung

Die vorliegende Erfindung betrifft topische Anwendung von substituierten Thiazolylamiden bei der Behandlung von Herpes-Erkrankungen beim Menschen, für die topische Anwendung geeignete Zubereitungen, sowie deren Herstellung.

Für die lokale Therapie von Herpes-Infektionen, insbesondere HSV 1 und HSV 2 werden in der Regel nukleosidische Wirkstoffe, beispielsweise Acyclovir eingesetzt. In vielen Fällen gelingt damit jedoch keine ausreichende Therapie der Herpes-Infektion.

Die vorliegende Erfindung betrifft topische anwendbare Zubereitungen, enthaltend 0,1 bis 99 Gew.-% einer Verbindung der allgemeinen Formel (I), 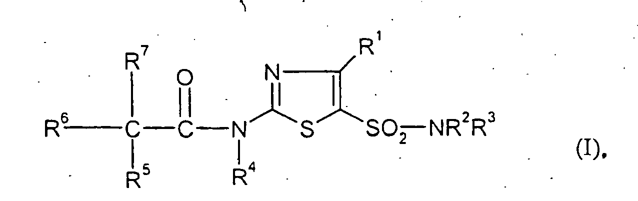 in welcher
- R¹: für Wasserstoff, Halogen, (C₁-C₆)-Alkyl, (C₁-C₆)-Alkoxy, Amino-(C₁-C₆)-alkyl oder Halogen-(C₁-C6)-alkyl steht,
- R² und R³: gleich oder verschieden sind und für Wasserstoff, (C₁-C₆)-Alkoxy, (C₃-C₈)-Cycloalkyl oder Biphenylaminocarbonyl stehen, oder
für (C₁-C₆)-Alkyl stehen, das gegebenenfalls durch 1 bis 3 Substituenten substituiert ist, die aus der Gruppe ausgewählt werden, die aus (C₃-C₆)-Cycloalkyl, (C₁-C₆)-Alkoxy, Halogen, Hydroxy, Amino, Tri-(C₁-C₆)-alkylsilyloxy, Resten der Formel 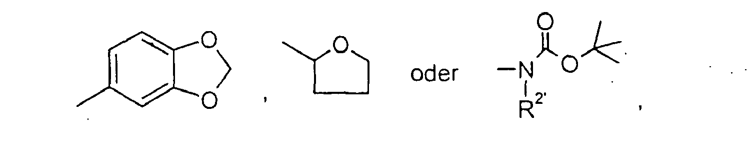
worin R^{2'} für Wasserstoff oder (C₁-C₄)-Alkyl steht,
einem 5- bis 6-gliedrigen aromatischen Heterocyclus mit bis zu 3 Heteroatomen aus der Reihe S, N und/oder O, wobei ein stickstoffhaltiger Heterocyclus auch über das Stickstoffatom gebunden sein kann,
einem gegebenenfalls über ein Stickstoffatom gebundenen 3- bis 8-gliedrigen gesättigten oder ungesättigten, nicht aromatischen, Heterocyclus mit bis zu 3 Heteroatomen aus der Reihe S, N und/oder O, und
(C₆-C₁₀)-Aryl, das seinerseits durch Hydroxy oder (C₁-C₆)-Alkoxy substituiert sein kann, besteht, oder
für eine Gruppe der Formel 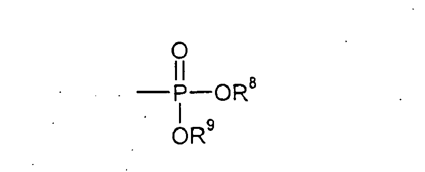 stehen, worin R⁸ und R⁹ gleich oder verschieden voneinander sind und für Wasserstoff und (C₁-C₄)-Alkyl stehen, oder
für eine Gruppe der Formel 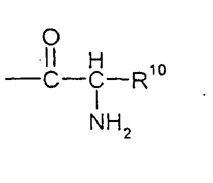 stehen; worin R¹⁰ die Seitengruppe einer natürlich vorkommenden α-Aminosäure darstellt, oder
für eine Gruppe der Formel 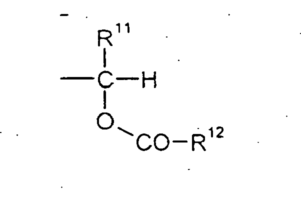 steht, worin R¹¹ für (C₁-C₄)-Alkyl steht, und R¹² für Wasserstoff, (C₁-C₄)Alkyl oder für eine Gruppe der Formel 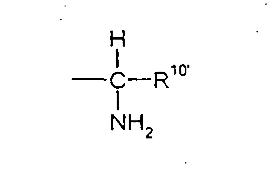 steht, worin R¹⁰ die Seitengruppe einer natürlich vorkommenden α-Aminosäure darstellt, oder
- R² und R³: gemeinsam mit dem Stickstoffatom einen 5- bis 6-gliedrigen gesättigten Heterocyclus bildet, der gegebenenfalls noch ein Sauerstoffatom aufweisen kann,
- R⁴: für Wasserstoff, (C₁-C₆)-Acyl, (C₂-C₆)-Alkenyl, (C₃-C₈)-Cycloalkyl steht, oder
- R⁴: für (C₁-C₆)-Alkyl steht, das gegebenenfalls substituiert sein kann durch 1 bis 3 Substituenten, die aus der Gruppe ausgewählt werden, die aus Halogen, Hydroxy, (C₃-C₈)-Cycloalkyl, (C₁-C₆)Acyl, (C₁-C₆)-Alkoxy, Carboxyl, 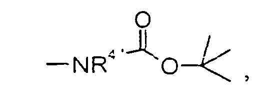 worin R4' für Wasserstoff steht,
-(OCH₂CH₂)ₙOCH₂CH₃, worin n 0 oder 1 ist, Phenoxy, (C₆-C₁₀)-Aryl und -NR¹³R¹⁴ besteht,
worin R¹³ und R¹⁴ gleich oder verschieden sind und Wasserstoff, (C₁-C₆)-Acyl, (C₁-C₆)-Alkyl, Carbamoyl, Mono- oder Di(C₁-C₆)-alkylamino(C₁-C₆)-alkyl, Mono- oder Di(C₁-C₆)-alkylaminocarbonyl, (C₆-C₁₀)-Aryl oder (C₁C₆)-Alkoxycarbonyl bedeuten, oder
R¹³ und R¹⁴ gemeinsam mit dem Stickstoffatom einen 5- bis 6-gliedrigen gesättigten Heterocyclus bilden, der gegebenenfalls ein weiteres Heteroatom aus der Reihe S oder O oder einen Rest der Formel -NR¹⁵ enthalten kann, und durch Oxo substituiert sein kann,
worin
R¹⁵ Wasserstoff oder (C₁-C₄)-Alkyl bedeutet, oder
- R⁴: für (C₁-C₆)-Alkyl steht, das durch einen 5- bis 6-gliedrigen aromatischen, gegebenenfalls benzokondensierten Heterocyclus mit bis zu 3 Heteroatomen aus der Reihe S, N und/oder O substituiert ist, wobei ein stickstoffhaltiger Heterocyclus auch über das Stickstoffatom gebunden sein kann, oder durch
Reste der Formeln 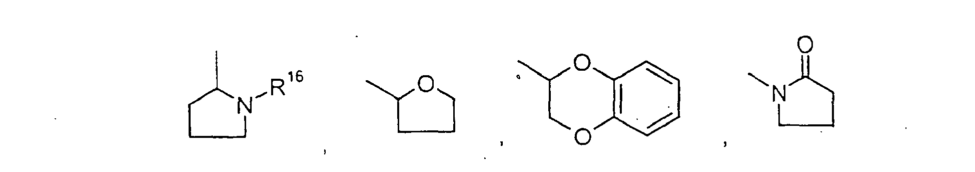 oder 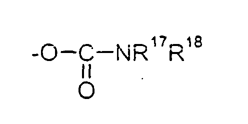 substituiert ist,
worin
- R¹⁶: Wasserstoff oder (C₁-C₆)-Alkyl bedeutet,
- R¹⁷ und R¹⁸: gleich oder verschieden sind und Wasserstoff, (C₁-C₆) Alkyl oder (C₆-C₁₀)-Aryl bedeuten, wobei zuvor genanntes (C₁-C₆)-Alkyl und (C₆-C₁₀)-Aryl gegebenenfalls durch 1 bis 3 Substituenten substituiert sein können, die aus der Gruppe ausgewählt werden, die aus Hydroxy, (C₁-C₆)-Alkoxy und Halogen besteht,
- R⁵: für Wasserstoff, (C₁-C₆)-Alkyl, Halogen, Amino, Mono- oder Di(C₁-C₆)-Alkylamino oder für (C₁-C₆)-Alkanoylamino steht,
- R⁶: für Phenyl steht, das gegebenenfalls mit ein bis drei Substituenten substituiert sein kann, die aus der Gruppe ausgewählt werden, die aus
- Halogen,
- (C₆-C₁₀)-Aryl, das gegebenenfalls durch 1 bis 3 Substituenten, ausgewählt aus (C₁-C₆)-Alkanoyl, (C₁-C₆)-Alkoxy, (C₁-C₆)-Alkyl, Halogen, (C₁-C₆)-Alkoxycarbonyl, Nitro, Halogen-(C₁-C₆)-lkyl, Halogen-(C₁C₆)-alkoxy, Amino, (C₁-C₆)-Alkylthio, Hydroxy, Carboxyl, Carbamoyl, Mono- oder Di-(C₁-C₆)-alkylaminocarbonyl, Mono- oder Di-(C₁-C₆)-alkanoylamino, (C₁-C₆)-Alkoxycarbonylamino, (C₁-C₆)-Alkylsulfoxy, (C₁-C₆)-Alkylsulfonyl, Tri-(C₁-C₆)-alkylsilyloxy, einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
- (C₁-C₆)-Alkoxy,
- (C₁-C₆)-Alkoxycarbonyl,
- (C₁-C₆)-Alkylthio,
- Hydroxy,
- Carboxyl,
- partiell fluoriertem (C₁-C₆)-Alkoxy mit bis zu 6 Fluoratomen,
- (C₁-C₆)-Alkyl, das gegebenenfalls durch einen Rest der Formeln 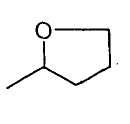 substituiert ist,
- einem gegebenenfalls über ein Stickstoffatom gebundenen 5- bis 6-gliedrigen aromatischen Heterocyclus mit bis zu 3 Heteroatomen aus der Reihe S, N und/oder O, der gegebenenfalls durch 1 bis 3 Substituenten, ausgewählt aus (C₁-C₆)-Alkanoyl, (C₁-C₆)-Alkoxy, (C₁-C₆)-Alkyl, Halogen, (C₁-C₆)-Alkoxycarbonyl, Nitro, Halogen-(C₁-C₆)-alkyl, Halogen-(C₁-C₆)-alkoxy, Amino, (C₁-C₆)-Alkylthio, Hydroxy, Carboxyl, Carbamoyl, Aminocarbonyl, Mono- oder Di-(C₁-C₆)-alkylaminocarbonyl, Mono- oder Di-(C₁-C₆)-alkanoylamino, (C₁-C₆)-Alkoxycarbonylamino, (C₁-C₆)-Alkylsulfoxy, (C₁-C₆)-Alkylsulfonyl, einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
- einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, der gegebenenfalls durch 1 bis 3 Substituenten ausgewählt aus Oxo, Halogen, Hydroxy, (C₁-C₆)-Alkoxycarbonyl, (C₁-C₆)-Alkoxycarbonylamino, (C₁-C₆)-Alkyl, Halogen-(C₁-C₆)-alkyl und Hydroxy-(C₁-C₆)-alkyl substituiert sein kann,
- (C₂-C₆)-Alkenyl
   und Gruppen der Formeln
   - -OR¹⁹,
   - -NR²⁰R²¹ oder -CO-NR²²R²³
   - Carbazol, Dibenzofuran oder Dibenzothiophen,
   - Xanthen oder 9,10-Dihydroacridin
   besteht,
   worin R¹⁹ Phenyl bedeutet, das seinerseits gegebenenfalls durch eine Gruppe der Formel -NR²⁴R²⁵ substituiert ist,
   worin
   R²⁴ und R²⁵ gleich oder verschieden sind und Wasserstoff, (C₁-C₆)-Alkyl oder (C₁-C₆)-Acyl bedeuten,
   oder
   R¹⁹ (C₁-C₆)-Alkyl bedeutet, das gegebenenfalls ein- bis dreifach durch Hydroxy und/oder Halogen substituiert ist;
   R²⁰ und R²¹ gleich oder verschieden sind und Wasserstoff, Carbamoyl, Mono- oder Di-(C₁-C₆)-alkylaminocarbonyl, Phenyl, (C₁-C₆)-Acyl oder (C₁-C₆)-Alkyl bedeuten, wobei zuvor genanntes (C₁-C₆)-Alkyl gegebenenfalls durch (C₁-C₆)-Alkoxy, (C₁-C₆)-Acyl, durch Phenyl oder durch einen 5- bis 6-gliedri- gen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
R²² und R²³ gleich oder verschieden sind und Wasserstoff oder (C₁-C₆)-Alkyl bedeuten,
- und R⁷: die Bedeutung von R⁵ aufweisen kann und mit dieser gleich oder verschieden sein kann,
und deren Salze, wobei die Zubereitung ferner zumindest einen weiteren Inhaltsstoff aufweist, der ausgewählt ist aus der Gruppe bestehend aus Augentropfen enthaltend 14 mg Polyvinylalkohol und 5,25 mg Chlorobutanol 1/2 H₂O pro 1 ml, sowie Natriumchlorid, einer Mischung aus 30 Gew.-% Isopropylmyristat und 70 Gew.-% Ethanol, einer Wollwachsalkoholsalbe bestehend aus 0,5 Gewichtsteilen Cetylstearylalkohol, 6 Gewichtsteilen Wollwachsalkohol und 93,5 Gewichtsteilen weißem Vaselin und einem Polyacrylatgel, wobei im Fall der Augentropfen sowie der Mischung aus Isopropylmyristol und Ethanol 2 Gew.-% der Verbindung enthalten sind.

Die Verbindungen können in den topisch anwendbaren Zubereitungen als solche oder als Salz mit einer Säure oder Base angewendet werden.

Physiologisch unbedenkliche Salze der erfindungsgemäßen Verbindungen können beispielsweise Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können weiterhin Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methylpiperidin.

Die erfindungsgemäßen Verbindungen können in Abhängigkeit von dem Substitutionsmuster in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweilige Mischungen. Die Racemformen lassen sich ebenso wie die Diastereomeren in bekannter Weise in die stereoisomer einheitlichen Bestandteile trennen.

(C₁-C₆)-Alkyl steht zweckmäßig für einen geradkettigen oder verzweigten Alkylrest mit 1 bis 6 Kohlenstoffatomen. Bevorzugt ist ein geradkettiger oder verzweigter Alkylrest mit 1 bis 4 Kohlenstoffatomen-(C₁-C₄). Beispielsweise seien genannt: Methyl, Ethyl, n-Propyl, Isopropyl, n-Butyl, sek.-Butyl, tert.-Butyl, n-Pentyl und n-Hexyl. Besonders bevorzugt ist ein geradkettiger oder verzweigter Alkylrest mit 1 bis 3 Kohlenstoffatomen ((C₁-C₃)-Alkyl).

Halogen(C1-C₆)-alkyl steht zweckmäßig für eine (C₁-C₆)-Alkylgruppe, die wie oben definiert sein kann, und die 1 bis 3 Halogenatome, nämlich F, Cl, Br und/oder I, bevorzugt Chlor oder Fluor, als Substituenten aufweist, beispielsweise seien erwähnt Trifluormethyl, Fluormethyl etc.

Hydroxy(C₁-C₆)-alkyl steht zweckmäßig für eine (C₁-C₆)-Alkylgruppe, die wie oben definiert sein kann, und die 1 bis 3 Hydroxygruppen als Substituenten aufweist, beispielsweise seien erwähnt Hydroxymethyl etc.

(C₂-C₆)-Alkenyl steht im Rahmen der Erfindung zweckmäßig für einen geradkettigen oder verzweigten Alkenylrest mit 2 bis 6 Kohlenstoffatomen. Beispielsweise seien genannt: Ethenyl, n-Prop-2-en-1-yl und n-But-2-en-1-yl. Bevorzugt ist ein geradkettiger oder verzweigter Alkenylrest mit 2 bis 4 Kohlenstoffatomen.

(C₁-C₆)-Alkoxy steht zweckmäßig für einen geradkettigen oder verzweigten Alkoxyrest mit 1 bis 6 Kohlenstoffatomen. Bevorzugt ist ein geradkettiger oder verzweigter Alkoxyrest mit 1 bis 4 Kohlenstoffatomen-(C₁-C₄). Beispielsweise seien genannt: Methoxy, Ethoxy, n-Propoxy, Isopropoxy, tert.-Butoxy, n-Pentoxy und n-Hexoxy. Besonders bevorzugt ist ein geradkettiger oder verzweigter Alkoxyrest mit 1 bis 3 Kohlenstoffatomen-(C₁-C₃).

Halogen-(C₁-C₆)-alkoxy steht zweckmäßig für einfach oder mehrfach halogensubstituiertes (C₁-C₆)-Alkoxy. Bezüglich des (C₁-C₆)-Alkoxy-Anteils sowie der Definition von Halogen sei auf die obige Definition verwiesen. Beispielsweise schließt Halogen-(C₁-C₆)-alkoxy ein oder mehrfach partiell chloriertes und/oder fluoriertes oder perfluoriertes (C₁-C₆)Alkoxy ein wie Trifluormethoxy, Fluormethoxy, Chlormethoxy, Pentafluorethoxy, Trifluormethylmethoxy etc. ein.

Partiell fluoriertes (C₁-C₆)-Alkoxy mit bis zu 6 Fluoratomen steht zweckmäßig für einen geradkettigen oder verzweigten Alkoxyrest mit 1 bis 6 Kohlenstoffatomen, der mit 1 bis 6, bevorzugt 1 bis 4, noch bevorzugter 1 bis 3 Fluoratomen substituiert sein kann. Bevorzugt ist ein geradkettiger oder verzweigter Alkoxyrest mit 1 bis 4 Kohlenstoffatomen und 1 bis 4 Fluoratomen. Beispielsweise seien genannt: Methoxy, Ethoxy, n-Propoxy, Isopropoxy, tert.-Butoxy, n-Pentoxy und n-Hexoxy, die jeweils ein bis 4 Fluoratome aufweisen. Besonders bevorzugt sind (1,3-Difluorprop-2-yl)-oxy und 1,1,2,2-Tetrafluorethoxy.

(C₁-C₆-Alkylthio steht zweckmäßig für einen geradkettigen oder verzweigten Alkylthiorest mit 1 bis 6 Kohlenstoffatomen. Bevorzugt ist ein geradkettiger oder verzweigter Alkylthiorest mit 1 bis 4 Kohlenstoffatomen-(C₁-C₄). Beispielsweise seien genannt: Methylthio, Ethylthio, n-Propylthio, Isopropylthio, tert.-Butylthio, n-Pentylthio und n-Hexylthio. Besonders bevorzugt ist ein geradkettiger oder verzweigter Alkylthiorest mit 1 bis 3 Kohlenstoffatomen (C₁-C₃)-Alkylthio.

(C₁-C₆)-Alkoxycarbonyl steht zweckmäßig für einen geradkettigen oder verzweigten Alkoxycarbonylrest mit 1 bis 6 Kohlenstoffatomen. Bevorzugt ist ein geradkettiger oder verzweigter Alkoxycarbonylrest mit 1 bis 4 Kohlenstoffatomen-(C₁-C₄). Beispielsweise seien genannt: Methoxycarbonyl, Ethoxycarbonyl, n-Propoxycarbonyl, Isopropoxycarbonyl und tert.-Butoxycarbonyl. Besonders bevorzugt ist ein geradkettiger oder verzweigter Alkoxycarbonylrest mit 1 bis 4 Kohlenstoffatomen-(C₁-C₄).

Mono- oder Di-(C₁-C₆)-alkylaminocarbonyl zweckmäßig steht im Rahmen der Erfindung zweckmäßig für eine Carbamoylgruppe (H₂N-CO-), in der ein oder beide Wasserstoffatome durch eine (C₁-C₆)-Alkylgruppe ersetzt sind. Bezüglich der Definition der (C₁-C₆)-Alkylgruppe sei auf die obigen Erläuterung von (C₁-C₆)-Alkyl verwiesen. Beispielsweise seien erwähnt Methylaminocarbonyl, Dimethylaminocarbonyl etc.

Mono- oder Di- (C₁-C₆)-acylamino steht im Rahmen der Erfindung zweckmäßig für eine Aminogruppe (H₂N-), in der ein oder beide Wasserstoffatome durch eine (C₁-C₆)-Acylgruppe ersetzt sind. Bezüglich der Definition der (C₁-C₆)-Acylgruppe sei auf die obigen Erläuterung von (C₁-C₆)Acyl verwiesen. Beispielsweise seien erwähnt (C₁-C₆)Alkanoyl, wie in der Definition von (C₁-C₆)Acyl erwähnt.

(C₁-C₆)-Alkylsulfoxy stellt zweckmäßig eine (C₁-C₆)-Alkyl-S(=O)-Gruppe dar, wobei bezüglich der (C₁-C₆)-Alkylgruppe auf die diesbezügliche obige Definition verwiesen werden kann.

(C₁-C₆₎-Alkylsulfonyl stellt zweckmäßig eine (C₁-C₆)-Alkyl-SO₂-Gruppe dar, wobei bezüglich der (C₁-C₆)-Alkylgruppe auf die diesbezügliche obige Definition verwiesen werden kann.

(C₆-C₁₀)-Aryl steht im allgemeinen für einen aromatischen.Rest mit 6 bis 10 Kohlenstoffatomen. Bevorzugte Arylreste sind Phenyl und Naphthyl.

(C₁-C₆-Acyl steht im Rahmen der Erfindung zweckmäßig für einen geradkettigen oder verzweigten Acylrest mit 1 bis 6 Kohlenstoffatomen. Beispielsweise seien genannt: Formyl, Acetyl, Ethanoyl, Propanoyl, Isopropanoyl, Butanoyl, Isobutanoyl und Pentanoyl. Bevorzugt ist ein geradkettiger oder verzweigter Acylrest mit 1 bis 4 Kohlenstoffatomen. Besonders bevorzugt sind Acetyl und Ethanoyl.

(C₃-C₈)-Cycloalkyl steht im Rahmen der Erfindung für Cyclopropyl, Cyclopentyl, Cyclobutyl, Cyclohexyl, Cycloheptyl oder Cyclooctyl. Bevorzugt seien genannt: Cyclopropyl, Cyclopentyl und Cyclohexyl. Die Bedeutung von (C₃-C₆)-Cycloalkyl steht entsprechend zweckmäßig für Cyclopropyl, Cyclopentyl, Cyclobutyl, Cyclohexyl.

Halogen steht im Rahmen der Erfindung im allgemeinen für Fluor, Chlor, Brom und Jod. Bevorzugt sind Fluor, Chlor und Brom. Besonders bevorzugt sind Fluor und Chlor.

(C₁-C₆)-Alkanoyl steht im Rahmen der Erfindung für Formyl sowie (C₁-C₅)-Alkylcarbonylgruppen, (C₁-C₅)-Alkyl eine geradkettige oder verzweigtkettige Alkylgruppe mit 1 bis 5 Kohlenstoffatomen sein kann, beispielsweise Acetyl, Propionyl, Butyryl, Pentanoyl.

Ein 5- bis 6-gliedriger aromatischer Heterocyclus mit bis zu 3 Heteroatomen aus der Reihe S, O und/oder N steht beispielsweise für Pyridyl, Pyrimidyl, Thienyl, Furyl, Pyrrolyl, Thiazolyl, N-Triazolyl, Oxazolyl oder Imidazolyl. Bevorzugt sind Pyridyl, Furyl, Thiazolyl und N-Triazolyl.

Ein 5- bis 6-gliedriger aromatischer benzokondensierter Heterocyclus mit bis zu 3 Heteroatomen aus der Reihe S, O und/oder N steht beispielsweise für Benzimidazolyl.

Ein 5- bis 6-gliedriger über ein Stickstoffatom gebundener gesättigter Heterocyclus, der aus zwei Substituentengruppen zusammen mit dem Stickstoffatom, an das sie gebunden sind, gebildet werden kann, und der gegebenenfalls ein weiteres Heteroatom aus der Reihe S oder O oder einem Rest der Formel -NR¹⁵, worin R¹⁵ wie oben definiert ist, enthalten kann, steht im Rahmen der Erfindung im allgemeinen für Morpholinyl, Piperidinyl, Piperazinyl, Methylpiperazinyl, Thiomorpholinyl oder Pyrrolidinyl Besonders bevorzugt sind Morpholinyl, Piperidinyl, Pyrrolidinyl und Thiomorpholinyl.

Ein gegebenenfalls über ein Stickstoffatom gebundener 3- bis 8-gliedriger gesättigter oder ungesättigter, nicht aromatischen Heterocyclus mit bis zu 3 Heteroatomen aus der Reihe S, N und/oder O schließt z.B. die oben genannten 5- bis 6-gliedrigen über ein Stickstoffatom gebundenen gesättigten Heterocyclen ein sowie 3-, 7- und 8-gliedrige Heterocyclen, wie z.B. Aziridine (z.B. 1-Azacyclopropan-1-yl), Azetidine (z.B. 1-Azacyclobutan-1-yl) und Azepine (z.B. 1-Azepan-1-yl) ein. Die ungesättigten Vertreter können 1 bis 2 Doppelbindungen im Ring enthalten.

Die Seitengruppe einer natürlich vorkommenden α-Aminosäure in der Bedeutung von R¹⁰ schließt beispielsweise ein: Wasserstoff (Glycin), Methyl (Alanin), Propan-2-yl (Valin), 2-Methyl-propan-1-yl (Leucin), 1-Methyl-propan-1-yl (Isoleucin), eine Propan-1,3-diyl-Gruppe, die mit dem Stickstoffatom der Aminogruppe verbunden ist (Prolin), eine 2-Hydroxypropan-1,3-diyl-Gruppe, die mit dem Stickstoffatom der Aminogruppe verbunden ist (Hydroxyprolin), eine Gruppe der Formel 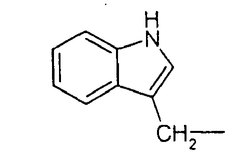 (Tryptophan), eine Benzylgruppe (Phenylalanin), eine Methylthioethylgruppe (Methionin), Hydroxymethyl (Serin), p-Hydroxybenzyl (Tyrosin), 1-Hydroxy-ethan-1-yl (Threonin), Mercaptomethyl (Cystein), Carbamoylmethyl (Asparagin), Carbamoylethyl (Glutamin), Carboxymethyl (Asparaginsäure), Carboxyethyl (Glutaminsäure), 4-Aminobutan-1-yl (Lysin), 3-Guanidinopropan-1-yl (Arginin), Imidazol-4-ylmethyl (Histidin), 3-Ureidopropan-1-yl (Citrullin), Mercaptoethyl (Homocystein), Hydroxyethyl (Homoserin), 4-Amino-3-hydroxy butan-1-yl (Hydroxylysin), 3-Amino-propan-1-yl (Omithin), etc.

Die Verbindungen werden in Form der topisch anwendbaren Zubereitungen zur Behandlung oder Prophylaxe von Infektionen und Erkrankungen der Haut mit Herpes, insbesondere Herpes Simplex, besonders HSV1 und HSV2, topisch appliziert. Auch eine Behandlung oder Prophylaxe tiefer gelegener oder systemischer Infektionen ist durch eine topische Applikation der Verbindungen bei ausreichender Bioverfügbarkeit möglich.

Die erfindungsgemäßen topisch anwendbaren Zubereitungen enthalten 0,1 bis 99, bevorzugt 0,5 bis 20 Gew.-% Wirkstoff der Formel (I). Besonders bevorzugt enthalten die erfindungsgemäßen topisch anwendbaren Zubereitungen 1 bis 5 Gew.-% Wirkstoff der Formel (I), insbesondere 2 bis 3 Gew.-% Wirkstoff.

In einer Ausführungsform betrifft die vorliegende Erfindung Suspensionen und Salben zur topischen Anwendung, die den Wirkstoff der Formel (I) enthalten.

Zu weiteren topischen Zubereitungen der Erfindung gehören Lösungen, Sprays, Lotionen, Gele, Cremes, Pasten, Emulsionen und Stifte, die den Wirkstoff der Formel (I), gegebenenfalls auch mehrere Wirkstoffe, enthalten.

Die topische Applikation der vorliegenden Formel (I) erfolgt auch in Form von Pflastern, Sprühpflastern, Okklusivverbänden, Umschlägen und gesteuerten Abgabesystemen. In diesen Zubereitungen können die Wirkstoffe in gelöster oder suspendierter Form enthalten sein.

Salben enthalten als Grundlage Kohlenwasserstoffgele, Lipogele, Absorptionsgrundlagen, W/O-Salbengrundlagen, Mischemulsionen oder Polyethylenglykole.

Cremes enthalten O/W-Grundlagen.

Pasten enthalten neben einer Salben- oder Cremegrundlage hohe Anteile pulverförmiger Bestandteile wie zum Beispiel Zinkoxid, Talk, Stärke oder Titandioxid.

Gele enthalten Lösungsmittel wie Wasser, Ethanol, Isopropanol oder Propylenglykol und werden unter Verwendung von Gelbildnern wie Celluloseethern, Alginaten, Polyacrylaten, Bentonit, Gelatine, Tragant, Polyvinylpyrrolidon oder Polyvinylalkohol hergestellt. Auch die Verwendung lipophiler Gelgrundlagen oder von Mikroemulsionen ist möglich.

Allen Zubereitungen können Stabilisatoren, Antioxidantien, Konservierungsmittel, Feuchthaltemittel, Rückfetter, Lösungsmittel oder Hilfsstoffe zur Verbesserung von Penetration und Wirksamkeit zugesetzt werden.

Beispiele von Penetrationsverbesserem sind Propylenglykol, Polyethylenglykol, Dimethylsulfoxid, Decylmethylsulfoxid, Azone, N-Methyl-pyrrolidon, Diethyltoluamid, Ethanol, Isopropylmyristat, Isopropylpalmitat, Ölsäure und seine Ester, mittelkettige Triglyceride, Dimethylisosorbit, 2-Octyldodecanol, verzweigtkettige Fettsäureester, Benzylalkohol, Harnstoff, Salicylate und Tenside.

In einer weiteren Ausführungsform betrifft die Erfindung Verbindungen der allgemeinen Formel (I) nach Anspruch 1: 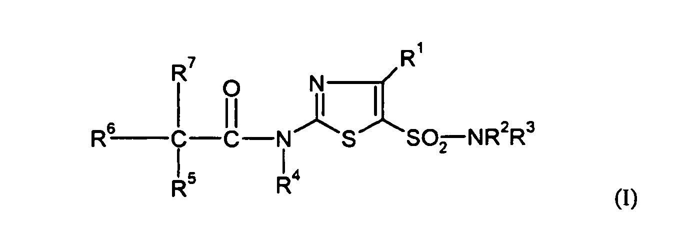 in welcher
- R¹: für Wasserstoff, Halogen, (C₁-C₆)-Alkyl, (C₁-C₆)-Alkoxy, Amino(C₁-C₆)alkyl oder Halogen(C₁-C₆)alkyl steht,
- R² und R³: gleich oder verschieden sind und für Wasserstoff, (C₁-C₆)-Alkoxy, (C₃-C₈)-Cycloalkyl oder Biphenylaminocarbonyl stehen, oder
für (C₁-C₆)-Alkyl stehen, das gegebenenfalls durch 1 bis 3 Substituenten substituiert ist, die aus der Gruppe ausgewählt werden, die aus (C₃-C₆)-Cycloalkyl, (C₁-C₆)-Alkoxy, Halogen, Hydroxy, Amino, Resten der Formel 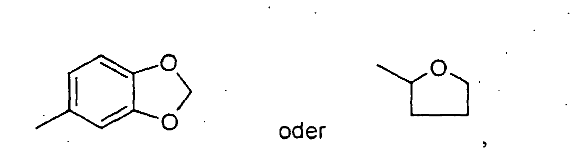 einem 5- bis 6-gliedrigen aromatischen Heterocyclus mit bis zu. 3 Heteroatomen aus der Reihe S, N und/oder O, wobei ein stickstoffhaltiger Heterocyclus auch über das Stickstoffatom gebunden sein kann,
einem gegebenenfalls über ein Stickstoffatom gebundenen 3- bis 8-gliedrigen gesättigten oder ungesättigten, nicht aromatischen, Heterocyclus mit bis zu 3 Heteroatomen aus der Reihe S, N und/oder O, und
(C₆-C₁₀)-Aryl, das seinerseits durch Hydroxy oder (C₁-C₆)-Alkoxy substituiert sein kann, besteht, oder
für eine Gruppe der Formel 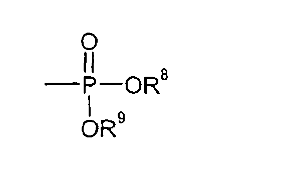 stehen, worin R⁸ und R⁹ gleich oder verschieden voneinander sind und für Wasserstoff und (C₁-C₄)-Alkyl stehen, oder
für eine Gruppe der Formel 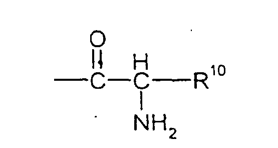 steht, worin R¹⁰ die Seitengruppe einer natürlich vorkommenden α-Aminosäure darstellt, oder
für eine Gruppe der Formel 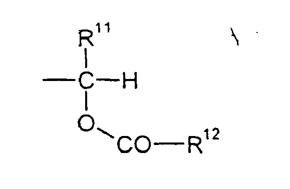 steht, worin R¹¹ für (C₁-C₄)-Alkyl steht, und R¹² für Wasserstoff, (C₁-C₄)-Alkyl oder für eine Gruppe der Formel 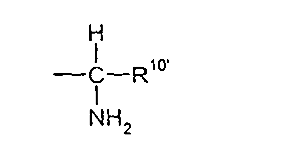 steht, worin R¹⁰ die Seitengruppe einer natürlich vorkommenden α-Aminosäure darstellt, oder
- R² und: R³ gemeinsam mit dem Stickstoffatom einen 5- bis 6-gliedrigen gesättigten Heterocyclus bildet, der gegebenenfalls noch ein Sauerstoffatom aufweisen kann,
- R⁴: für Wasserstoff, (C₁-C₆)-Acyl, (C₂-C₆)-Alkenyl, (C₃-C₈)-Cycloalkyl steht, oder
- R⁴: für (C₁-C₆)-Alkyl steht, das gegebenfalls substituiert sein kann durch 1 bis 3 Substituenten, die aus der Gruppe ausgewählt werden, die aus Halogen, Hydroxy, (C₁-C₆)Acyl, (C₁-C₆)Alkoxy,
-(OCH₂CH₂)ₙOCH₂CH₃, worin n 0 oder 1 ist, Phenoxy, (C₆-C₁₀)-Aryl und -NR¹³R¹⁴ besteht,
worin
R¹³ und R¹⁴ gleich oder verschieden sind und Wasserstoff, (C₁-C₆)-Acyl, (C₁-C₆)-Alkyl, Carbamoyl, Mono- oder Di(C₁-C₆)-alkylamino(C₁-C₆)alkyl, Mono- oder Di(C₁-C₆)-alkylaminocarbonyl, (C₆-C₁₀)-Aryl oder (C₁-C₆)-Alkoxycarbonyl bedeuten, oder
R¹³ und R¹⁴ gemeinsam mit dem Stickstoffatom einen 5- bis 6 gliedrigen gesättigten Heterocyclus bilden, der gegebenenfalls ein weiteres Heteroatom aus der Reihe S oder O oder einen Rest der Formel -NR¹⁵ enthalten kann, und durch Oxo substituiert sein kann,
worin R¹⁵ Wasserstoff oder (C₁-C₄)-Alkyl bedeutet, oder
- R⁴: für (C₁-C₆)-Alkyl steht, das durch einen 5- bis 6-gliedrigen aromatischen, gegebenenfalls benzokondensierten Heterocyclus mit bis zu 3 Heteroatomen aus der Reihe S, N und/oder O substituiert ist, wobei ein stickstoffhaltiger Heterocyclus auch über das Stickstoffatom gebunden sein kann, oder durch Reste der Formeln 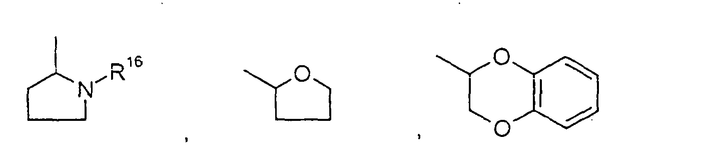 oder 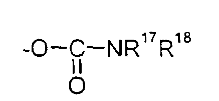 substituiert ist, worin
R¹⁶ Wasserstoff oder (C₁-C₆)-Alkyl bedeutet,
R¹⁷ und R¹⁸ gleich oder verschieden sind und Wasserstoff, (C₁-C₆) Alkyl oder (C₆-C₁₀)-Aryl bedeuten, wobei zuvor genanntes (C₁-C6)-Alkyl und (C₆-C₁₀)-Aryl gegebenenfalls durch 1 bis 3 Substituenten substituiert sein können, die aus der Gruppe ausgewählt werden, die aus Hydroxy, (C₁-C₆)-Alkoxy und Halogen besteht,
- R⁵: für Wasserstoff, (C₁-C₆)-Alkyl, Halogen, Amino, Mono- oder Di(C₁-C₆)-Alkylamino oder für (C₁-C₆)-Alkanoylamino steht,
- R⁶: für Phenyl steht, das gegebenenfalls mit ein bis drei Substituenten substituiert sein kann, die aus der Gruppe ausgewählt werden, die aus
- Halogen,
- (C₆-C₁₀)-Aryl, das gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di(C₁-C₆)alkylaminocarbonyl, Mono- oder Di(C₁C₆)alkanoylamino, (C₁-C₆)Alkoxycarbonylamino, (C₁-C₆)Alkylsulfoxy, (C₁-C₆)Alkylsulfonyl, Tri(C₁-C₆)alkylsilyloxy, einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
- (C₁-C₆)-Alkoxy,
- (C₁-C₆)-Alkoxycarbonyl,
- (C₁-C₆)-Alkylthio,
- Hydroxy,
- Carboxyl,
- partiell fluoriertem (C₁-C₆)-Alkoxy mit bis zu 6 Fluoratomen,
- (C₁-C₆)-Alkyl, das gegebenenfalls durch einen Rest der Formel 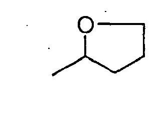 substituiert ist,
- einem gegebenenfalls über ein Stickstoffatom gebundenen 5- bis 6-gliedrigen aromatischen Heterocyclus mit bis zu 3 Heteroatomen aus der Reihe S, N und/oder O, der gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino, (C₁-C₆)Alkylthio, Hydroxy, Carboxyl, Carbamoyl, Mono- oder Di(C₁-C₆)alkylaminocarbonyl, Mono- oder Di(C₁-C₆)alkanoylamino, (C₁-C₆)Alkoxycarbonylamino, (C₁-C₆)Alkylsulfoxy, (C₁-C₆)Alkylsulfonyl, einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
- einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, der gegebenenfalls durch 1 bis 3 Substituenten ausgewählt aus Oxo, Halogen, Hydroxy, (C₁-C₆)-Alkoxycarbonyl, (C₁-C₆)-Alkoxycarbonylamino, (C₁-C₆)-Alkyl, Halogen(C₁-C₆)alkyl und Hydroxy(C₁-C₆)-alkyl substituiert sein kann,
   und Gruppen der Formeln
   --OR¹⁹,
   - -NR²⁰R²¹ oder -CO-NR²²R²)
      besteht,
      worin
      R¹⁹ Phenyl bedeutet, das seinerseits gegebenenfalls durch eine Gruppe der Formel -NR²⁴R²⁵ substituiert ist,
      worin
      R²⁴ und R²⁵ gleich oder verschieden sind und Wasserstoff, (C₁-C₆)-Alkyl oder (C₁-C₆)-Acyl bedeuten,
      oder
      R¹⁹ (C₁-C₆)-Alkyl bedeutet, das gegebenenfalls ein- bis dreifach durch Hydroxy und/oder Halogen substituiert ist,
- R²⁰ und R²¹: gleich oder verschieden sind und Wasserstoff, Carbamoyl, Mono- oder Di(C₁-C₆)alkylaminocarbonyl, Phenyl, (C₁-C₆)-Acyl oder (C₁-C₆)-Alkyl bedeuten,
wobei zuvor genanntes (C₁-C₆)-Alkyl gegebenenfalls durch (C₁-C₆)-Alkoxy, (C₁-C₆)-Acyl, durch Phenyl oder durch einen 5- bis 6-gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
- R²² und R²³: gleich oder verschieden sind und Wasserstoff oder (C₁-C₆)-Alkyl bedeuten, und
- R⁷: die Bedeutung von R⁵ aufweisen kann und mit dieser gleich oder verschieden sein kann,
und deren Salze.

In einer bevorzugten Ausführungsform betrifft die Erfindung Verbindungen der allgemeinen Formel (I) 1, worin R¹ für Wasserstoff oder (C₁-C₆)-Alkyl, insbesondere für Methyl steht.

In einer weiteren bevorzugten Ausführungsform betrifft die Erfindung Verbindungen der allgemeinen Formel (I), worin R² und R³ jeweils unabhängig für Wasserstoff, (C₁-C₆)-Alkyl oder 2-Hydroxyethyl stehen, insbesondere für Wasserstoff, Methyl und 2-Hydroxyethyl.

In einer weiteren bevorzugten Ausführungsform betrifft die Erfindung Verbindungen der allgemeinen Formel (I), worin R² für Wasserstoff und R³ für (C₁-C₆)-Alkoxy oder (C₃-C₈)-Cycloalkyl steht, oder für (C₁-C₆)-Alkyl steht, das gegebenenfalls durch 1 bis 3 Substituenten substituiert ist, die aus der Gruppe ausgewählt werden, die aus (C₃-C₆)-Cycloalkyl, (C₁-C₆)-Alkoxy, Halogen, Hydroxy, Amino, Tri-(C₁-C₆)-alkylsilyloxy,
einem 5- bis 6-gliedrigen aromatischen Heterocyclus mit bis zu 3 Heteroatomen aus der Reihe S, N und/oder O, wobei ein stickstoffhaltiger Heterocyclus auch über das Stickstoffatom gebunden sein kann,
einem gegebenenfalls über ein Stickstoffatom gebundenen 3- bis 8-gliedrigen gesättigten oder ungesättigten, nicht aromatischen, Heterocyclus mit bis zu 3 Heteroatomen aus der Reihe S, N und/oder O, und
(C₆-C₁₀)-Aryl, das seinerseits durch Hydroxy oder (C₁-C₆)-Alkoxy substituiert sein kann, besteht, oder
in einer weiteren bevorzugten Ausführungsform betrifft die Erfindung Verbindungen der allgemeinen Formel (I), worin R⁴ für Wasserstoff oder (C₁-C₆)-Alkyl, insbesondere Methyl steht.

In einer weiteren bevorzugten Ausführungsform betrifft die Erfindung Verbindungen der allgemeinen Formel (I), worin R⁵ für Wasserstoff steht.

In einer weiteren bevorzugten Ausführungsform betrifft die Erfindung Verbindungen der allgemeinen Formel (I), worin
- R⁶: für Phenyl steht, das gegebenenfalls mit ein bis drei Substituenten substituiert sein kann, die aus der Gruppe ausgewählt werden, die aus
- -: Halogen,
- -: (C₆-C₁₀)-Aryl, das gegebenenfalls durch 1 bis 3 Substituenten, ausgewählt aus (C₁-C₆)Alkanoyl, (C₁-C₆)-Alkoxy, (C₁-C₆)-Alkyl, Halogen, (C₁-C₆)Alkoxycarbonyl, Nitro, Halogen(C₁-C₆)alkyl, Halogen(C₁-C₆)alkoxy, Amino,
Hydroxy, Mono- oder Di(C₁-C₆)alkylamino, Mono- oder Di(C₁-C₆)-Alkanoylamino, (C₁-C₆)Alkoxycarbonylamino, und/oder Cyano substituiert sein kann, und
- einem gegebenenfalls über ein Stickstoffatom gebundenen 5- bis 6-gliedrigen aromatischen Heterocyclus mit bis zu 3 Heteroatomen aus der Reihe S, N und/oder O, der gegebenenfalls durch 1 bis 2 Halogenatome substituiert sein kann,
besteht.

Insbesondere steht R⁶ für 1,1'-Biphenyl-4-yl, 4-(2-Pyridinylphenyl) oder 4-(1H-pyrazol-1-yl)phenyl, wobei diese Reste mit 1 bis 2 Fluoratomen substituiert sein können.

In einer weiteren bevorzugten Ausführungsform betrifft die Erfindung Verbindungen, die die folgende Formel aufweisen: 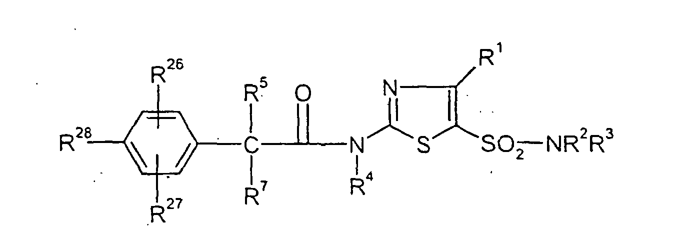 worin
- R¹, R², R³, R⁴, R⁵ und R⁷: wie im Anspruch 1 definiert sind,
- R²⁶ und R²⁷: gleich oder verschieden sind und für Wasserstoff, Halogen, (C₁-C₆)-Alkoxy, (C₁-C₆)-Alkoxycarbonyl, (C₁-C₆)-Alkylthio, Hydroxy, Carboxyl, partiell fluoriertes (C₁-C₆)-Alkoxy mit bis zu 6 Fluoratomen, (C₁-C₆)-Alkyl, eine Gruppe der Formeln -OR¹⁹, -NR²⁰R²¹ oder -CO-NR²²R²³ stehen, worin
- R¹⁹: Phenyl bedeutet, das seinerseits gegebenenfalls durch eine Gruppe der Formel -NR²⁴R²⁵ substituiert ist,
worin
- R²⁴ und R²⁵: gleich oder verschieden sind und Wasserstoff, (C₁-C₆)-Alkyl oder (C₁-C₆)-Acyl bedeuten, oder
- R¹⁹: (C₁-C₆)-Alkyl bedeutet, das gegebenenfalls ein- bis dreifach durch
Hydroxy und/oder Halogen substituiert ist,
- R²⁰ und R²¹: gleich oder verschieden sind und Wasserstoff, Carbamoyl, Mono- oder Di(C₁-C₆)alkylaminocarbonyl, Phenyl, (C₁-C₆)-Acyl oder (C₁-C₆)-Alkyl bedeuten,
wobei zuvor genanntes (C₁-C₆)-Alkyl gegebenenfalls durch (C₁-C₆)-Alkoxy, (C₁-C₆)-Acyl, Phenyl oder durch einen 5- bis 6-gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
- R²² und R²³: gleich oder verschieden sind und Wasserstoff oder (C₁-C₆)-Alkyl bedeuten,
- R²⁸: für (C₆-C₁₀)-Aryl steht, das gegebenenfalls durch 1 bis 3 Substituenten, ausgewählt aus (C₁-C₆)-Alkanoyl, (C₁-C₆)-Alkoxy, (C₁-C₆)-Alkyl, Halogen, (C₁-C₆)-Alkoxycarbonyl, Nitro, Halogen-(C₁-C₆)-alkyl, Halogen-(C₁-C₆)-alkoxy, Amino, (C₁-C₆)-Alkylthio, Hydroxy, Carboxyl, Carbamoyl, Mono- oder Di-(C₁-C₆)-alkylaminocarbonyl, Mono- oder Di-(C₁-C₆)-alkanoylamino, (C₁-C₆)-Alkoxycarbonylamino, (C₁-C₆)-Alkylsulfoxy, (C₁-C₆)-Alkylsulfonyl, Tri-(C₁-C₆)-alkylsilyloxy, einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann, oder
- R²⁸: für einen gegebenenfalls über ein Stickstoffatom gebundenen 5- bis 6- gliedrigen aromatischen Heterocyclus mit bis zu 3 Heteroatomen aus der Reihe S, N und/oder O steht, der gegebenenfalls durch 1 bis 3 Substituenten, ausgewählt aus (C₁-C₆)-Alkanoyl, (C₁-C₆)-Alkoxy, (C₁-C₆)-Alkyl, Halogen, (C₁-C₆)-Alkoxycarbonyl, Nitro, Halogen-(C₁-C₆)-alkyl, Halogen-C₁-C₆)-alkoxy, Amino, (C₁-C₆)-Alkylthio, Hydroxy, Carboxyl, Carbamoyl, Mono- oder Di-(C₁-C₆)-alkylaminocarbonyl, Mono- oder Di-(C₁-C₆)-alkanoylamino, (C₁-C₆)-Alkoxycarbonylamino, (C₁-C₆)-Alkylsulfoxy, (C₁-C₆)-Alkylsulfonyl, einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
und deren Salze.

Die Verbindungen der allgemeinen Formel (I) können hergestellt werden, indem man
[A] Verbindungen der allgemeinen Formel (II) 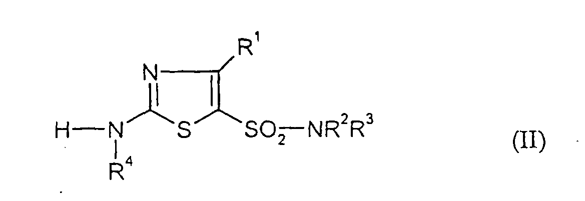 in welcher
   R¹, R², R³ und R⁴ die oben angegebene Bedeutung haben,
   mit Verbindungen der allgemeinen Formel (III) 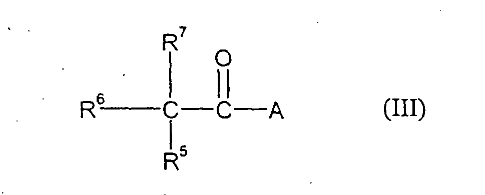 in welcher
   - A: für eine Abgangsgruppe, wie z.B. Halogen, vorzugsweise Chlor, oder Hydroxy steht, und R⁵, R⁶ und R⁷ die oben angegebene Bedeutung haben, in inerten Lösemitteln, gegebenenfalls in Anwesenheit einer Base und/oder eines Hilfsmittels zu Verbindungen der Formel (I) umsetzt,
[B] Verbindungen der allgemeinen Formel (IV) 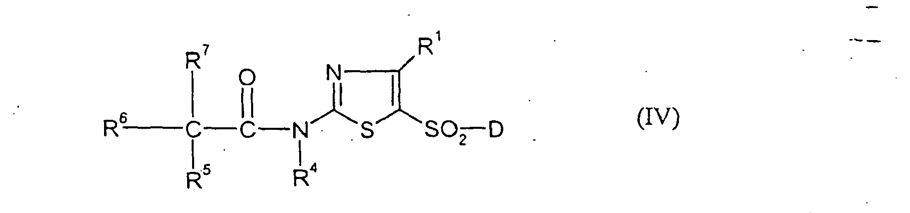 worin
   - R¹ R⁴, R⁵, R⁶ und R⁷: die oben angegebene Bedeutung haben, und D ein Halogenatom, vorzugsweise Chlor ist, mit Aminen der allgemeinen Formel (V):

   HNR²R³ (V)

   worin
   R² und R³ die oben angegebene Bedeutung haben, in inerten Lösemitteln zu Verbindungen der Formel (I) umsetzt,
[C] Verbindungen der allgemeinen Formel (X) 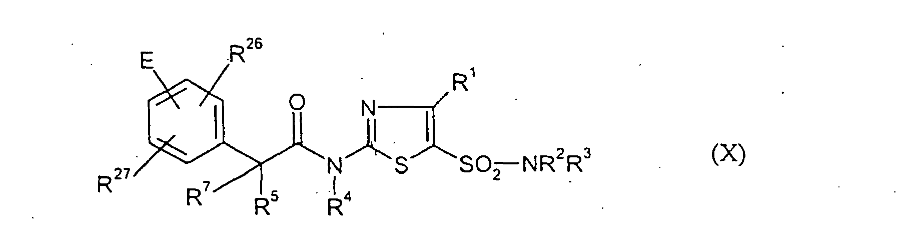 worin
   - R¹, R², R³, R⁴, R⁵, R⁷, R²⁶ und R²⁷: die oben angegebene Bedeutung haben, und E Trifluormethansulfonat oder Halogen, vorzugsweise Brom oder Iod ist, mit Boronsäuren oder Stannanen der allgemeinen Formel (XI):

   R²⁸M (XI)

   worin
   R²⁸ die oben angegebene Bedeutung hat und M beispielsweise eine Tri(C₁-C₆)alkylstannylgruppe, wie eine Trimethylstannylgruppe oder eine Boronsäuregruppe sein kann, in inerten Lösemitteln in Gegenwart von Palladiumkatalysatoren, z.B. Tetrakis(triphenylphosphan)palladium (0), ggf. in Anwesenheit von Base, z.B. Kaliumphosphat bei Temperaturen von 50-140°C zu Verbindungen der Formel (XIV) 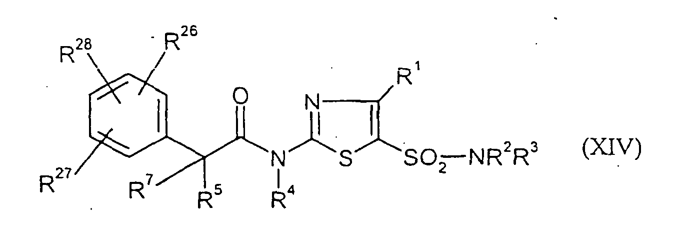 umsetzt, und
[D] Verbindungen der allgemeinen Formel (XII) 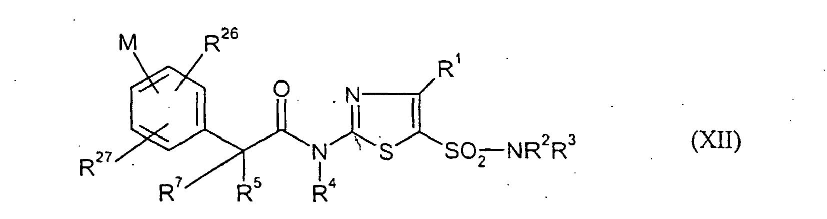
worin
- R¹, R², R³, R⁴, R⁵, R⁷, R²⁶ und R²⁷: die oben angegebene Bedeutung haben, und M die oben angegebene Bedeutung besitzt, mit Trifluormethansulfonaten oder Halogeniden der allgemeinen Formel (XIII):

R²⁸E (XIII)

worin
- R²⁸: die oben angegebene Bedeutung hat und E die oben angegebene Bedeutung besitzt, in inerten Lösemitteln in Gegenwart von Palladiumkatalysatoren, z.B. Tetrakis(triphenylphosphan)palladium (0), ggf. in Anwesenheit von Base, z.B. Kaliumphosphat bei Temperaturen von 50-140°C zu Verbindungen der Formel (XIV) umsetzt.

Das erfindungsgemäße Verfahren [A] kann durch folgendes Formelschemata beispielhaft erläutert werden:

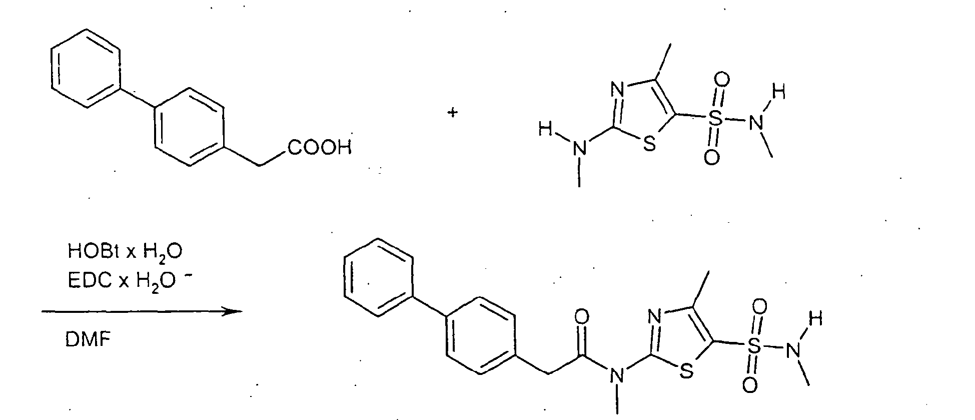

### Hierin bedeuten:

HOBt: 1-Hydroxy-1H-benzotriazol
EDC: *N'*-(3-Dimethylaminopropyl)-*N*-ethylcarbodiimid x HCl
DMF: *N*,*N*-Dimethylformamid

Das erfindungsgemäße Verfahren [C] kann durch folgendes Formelschemata beispielhaft erläutert werden:

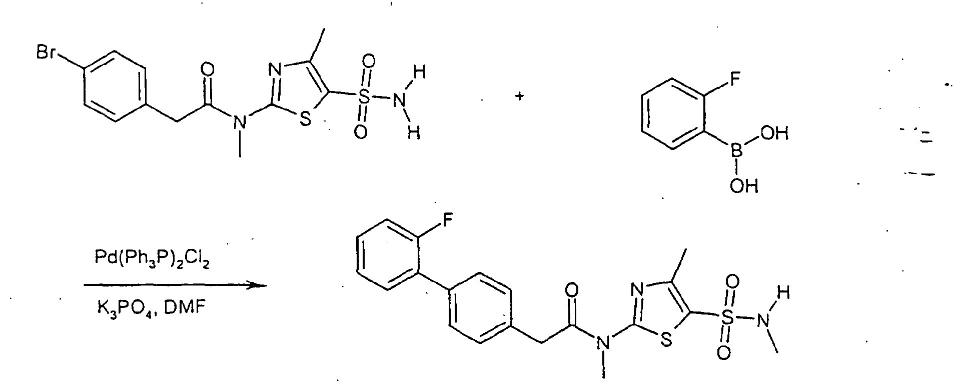

### Hierin bedeutet:

DMF: *N,N*-Dimethylformamid

Das erfindungsgemäße Verfahren [D] kann durch folgendes Förmelschemata beispielhaft erläutert werden:

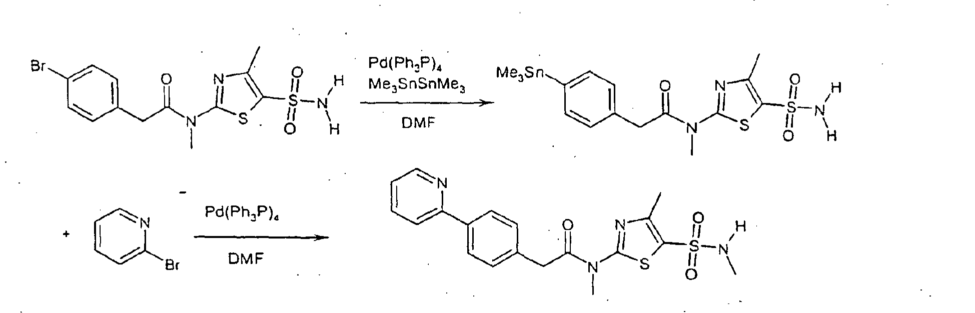

### Hierin bedeutet:

DMF: *N,N*-Dimethylformamid

Als Lösemittel für die Verfahren [A], [B], [C] und [D] eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Dimethylsulfoxid, Dimethylformamid (DMF) oder Acetonitril. Ebenso ist es möglich, Gemische der genannten Lösemittel zu verwenden. Bevorzugt ist DMF.

Als Basen für das erfindungsgemäße Verfahren [A] können im allgemeinen anorganische oder organische Basen eingesetzt werden. Hierzu gehören vorzugsweise organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, N-Methylmorpholin oder N-Methylpiperidin oder Morpholin. Bevorzugt ist Triethylamin.

Als Hilfsmittel eignen sich an sich bekannte Dehydratisierungs- bzw. Kupplungsreagenzien, wie beispielsweise Carbodiimide, wie Diisopropylcarbodiimid, Dicyclohexylcarbodiimid (DCC) oder *N*-(3-Dimethylaminopropyl)-*N*'-ethylcarbodiimid (EDC), oder Carbonylverbindungen wie Carbonyldiimidazol (CDI) oder Isobutylchloroformiat, oder 1,2-Oxazoliumverbindungen wie 2-Ethyl-5-phenyl-1,2-oxazolium-3-sulfonat, oder Phosphorverbindungen wie Propanphosphonsäureanhydrid, Phosphorsäurediphenylesterazid, Benzotriazolyl-*N*-oxy-tris(dimethylamino)phosphonium-Hexafluorophosphat (BOP), oder Uronium-Verbindungen wie *O*-Benzotriazol-1-yl-*N,N,N',N'*-tetramethyluronium-Hexafluoro-phosphat (HBTU), oder Methansulfonsäurechlorid, gegebenenfalls in Gegenwart von Hilfsstoffen wie N-Hydroxysuccinimid oder N-Hydroxybenzotriazol.

Im allgemeinen setzt man die Base in einer Menge von 0,05 Mol bis 10 Mol, bevorzugt von 1 Mol bis 2 Mol bezogen auf 1 Mol der Verbindung der Formel (III) ein.

Die erfindungsgemäßen Verfahren werden im allgemeinen in einem Temperaturbereich von -50°C bis +100°C, bevorzugt von -30°C bis +60°C, durchgeführt.

Die erfindungsgemäßen Verfahren werden im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel (II) können beispielsweise hergestellt werden, indem man Verbindungen der allgemeinen Formel (VI) 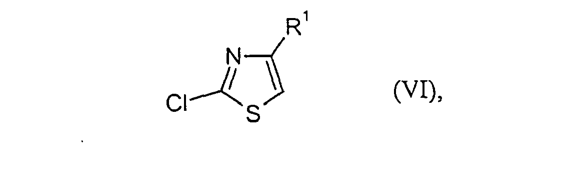 in welcher
- R¹: die oben angegebene Bedeutung hat,
durch Umsetzung mit dem System Chlorsulfonsäure/SOCl₂ in die Verbindungen der allgemeinen Formel (VII) 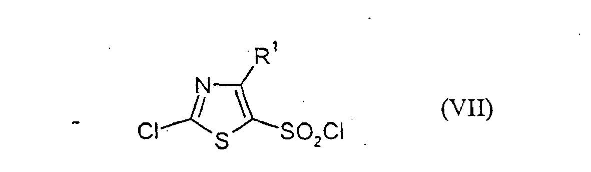 in welcher
- R¹: die oben angegebene Bedeutung hat,
überführt, anschließend mit Aminen der allgemeinen Formel (V)

HNR²R³ (V)

in welcher
- R² und R³: die oben angegebene Bedeutung haben,
in inerten Lösemitteln die Verbindungen der allgemeinen Formel (VIII) 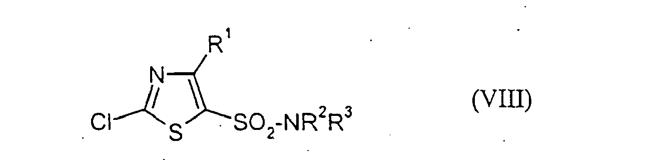 in welcher
- R¹, R² und R³: die oben angegebene Bedeutung haben,
herstellt, und in einem letzten Schritt eine Umsetzung mit Aminen der allgemeinen Formel (IX)

H₂N-R^{4'} (IX)

in welcher
- R^{4'}: die oben angegebene Bedeutung von R⁴ hat und mit dieser gleich oder verschieden ist, aber nicht Wasserstoff ist,
in inerten Lösemitteln und in Anwesenheit einer Base durchführt.

Die Reaktion mit Chlorsulfonsäure/SO₂Cl erfolgt zunächst bei Raumtemperatur und anschließend unter der Rückflusstemperatur des jeweiligen Ethers.

Die Umsetzung wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Lösemittel für die Umsetzung mit den Aminen der allgemeinen Formel (V) eignen sich Alkohole wie beispielsweise Methanol, Ethanol, Propanol und Isopropanol. Bevorzugt ist Methanol.

Die Umsetzung mit den Aminen der allgemeinen Formel (V) erfolgt zunächst bei Raumtemperatur und anschließend unter der Rückflusstemperatur des jeweiligen Ethers.

Die Umsetzung wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Umsetzung mit den Verbindungen der allgemeinen Formel (IX) erfolgt in Ethern wie beispielsweise Diethylether, Dioxan, Tetrahydrofuran oder Glykoldimethylether. Bevorzugt ist Methanol.

Als Basen können im allgemeinen anorganische oder organische Basen eingesetzt werden. Hierzu gehören vorzugsweise organische Amine (Tri(C₁-C₆)alkylamine, wie Triethylamin), oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Bevorzugt ist Triethylamin.

Im allgemeinen setzt man die Base in einer Menge von 0,05 Mol bis 10 Mol, bevorzugt von 1 Mol bis 2 Mol bezogen auf 1 Mol der Verbindung der Formel (VIII) ein.

Die Verbindungen der allgemeinen Formel (VI) sind teilweise bekannt oder nach üblichen Methoden herstellbar [vgl. Hantzsch, Chem. Ber. 1927, 60, 2544].

Die Verbindungen der allgemeinen Formel (VII) und (VIII) sind neu und können wie oben beschrieben hergestellt werden.

Amine der allgemeinen Formeln (V) und (IX) sind bekannt.

Verbindungen der allgemeinen Formeln (III) sind bekannt oder lassen sich nach literaturbekannten Verfahren herstellen.

Biphenylmethylcarbonsäure- bzw. Biphenylessigsäurederivate der Formel (III) lassen sich in an sich bekannter Weise durch übergangsmetallkatalysierte, beispielsweise palladiumkatalysierte Kupplungsreaktionen, wie z.B. der Suzuki- oder Stille-Kupplung herstellen. Die Pyridylphenylmethylcarbonsäurederivate der Formel (III) sind literaturbekannt (siehe z.B. M. Artico et al. in Eur. J. Med. Chem. (1992) 27, 219-228) oder lassen sich nach an sich bekannten Verfahren herstellen. Die folgenden Reaktionsschemata A, B, C und D illustrieren beispielhaft die Synthese von Biphenylessigsäurederivaten aus den entsprechenden Boronsäuren sowie die Synthese von Pyridylphenylessigsäurederivaten aus den entsprechenden Stannylverbindungen:

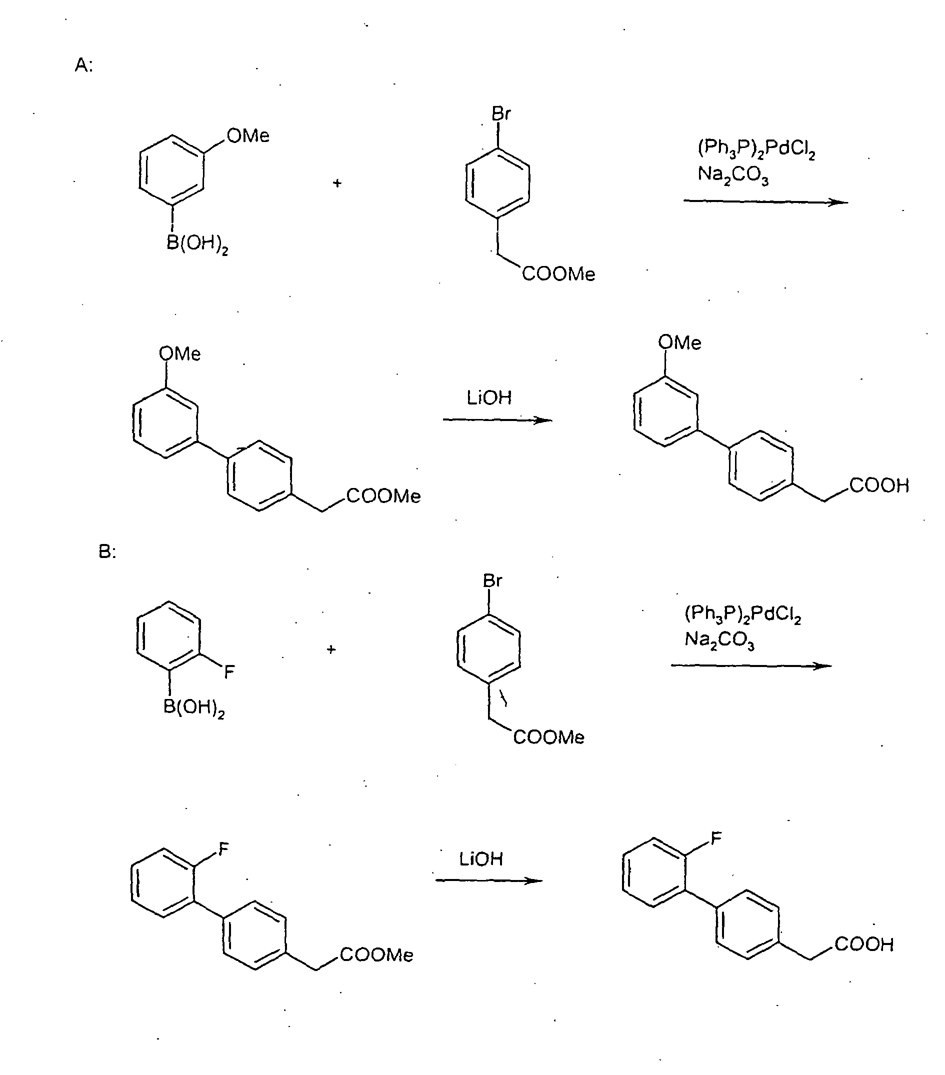

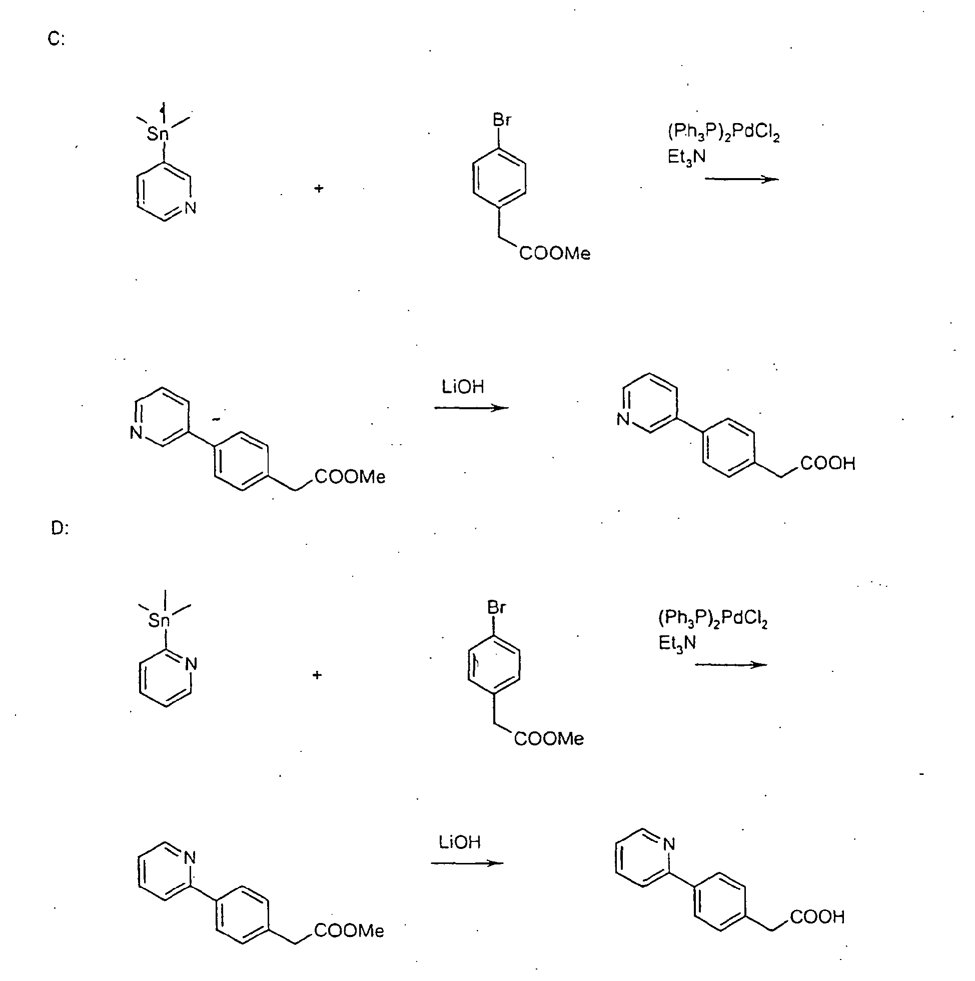

Verbindungen der Formel (III), in denen R⁵ und R⁷ zum Beispiel Fluor ist, lassen sich nach dem im folgenden Reaktionsschema gezeigten Verfahren herstellen:

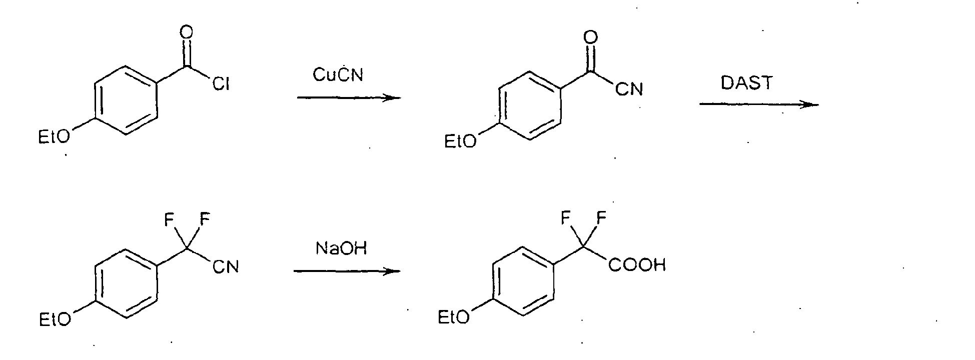

Die Fluorierung mit DAST (N,N-Diethylaminoschwefeltrifluorid) erfolgt dabei gemäß J. Fluor. Chem. 61, 1993, 117.

Die vorliegende Erfindung betrifft daher substituierte Thiazolylamide zur topischen Anwendung. In einer weiteren Ausführungsform betrifft die vorliegende Erfindung die topische Anwendung an Haut, Auge oder Vagina. Als gut geeignet haben sich wasserfreie Zubereitungen erwiesen, insbesondere wasserfreie und nicht fettende Zubereitungen. Besonders bevorzugt sind die noch näher bezeichneten "PEG-Isogele". Es hat sich gezeigt, das mit diesen Zubereitungen auch eine Herpes-Prophylaxe im Genitalbereich erzielt werden kann.

### Formulierung der Wirkstoffe

### Ophthalmologische topische Anwendung

Die Wirkstoffe werden zur Bildung einer 2 Gew-% Suspension mit Liquifilm^{©} Augentropfen (Allergan, Ettlingen, Deutschland) (1 ml enthaltend Polyvinylalkohol 14 mg, sowie als Hilfsstoff Chlorobutanol ½ H_{2O} 5,25 mg , Natriumchlorid) versetzt und in einem Glasröhrchen 15min in einem Ultraschallbad bei 15°C sonifiziert.

### Dermatologische topische Anwendung

Die Beispiele für Zubereitungen, die PEG-Isogele enthalten, fallen nicht in den Schutzbereich der vorliegenden Ansprüche

### 1) Alkoholische Suspension

Die Wirkstoffe werden zur Bildung einer 2 Gew.-% Suspension mit 30 Gew.-% Isopropylmyristat/70 Gew.-% Ethanol versetzt und in einem Glasröhrchen 15 min in einem Ultraschallbad bei 15°C sonifiziert.

### 2) Streichfähige Zubereitungen

Zur Herstellung der Salbenzubereitung wird Wollwachsalkoholsalbe nach DAB 9 eingesetzt. Der Wirkstoff wird in einer aufgeschmolzenen Mischung aus 0.5 Gewichtsteilen Cetylstearylalkohol (Qualität nach DAB 9), 6 Gewichtsteilen Wollwachsalkohol (Qualität nach DAB 9) und 93.5 Gewichtsteilen weißem Vaselin (Qualität nach DAB 9) suspendiert. Die Mischung wird bis zum Abkühlen auf Raumtemperatur (etwa 21°C) gerührt.

Zur Herstellung einer sogenannten "PEG-Isogel" Zubereitung wird der Wirkstoff in der Schmelze eine Mischung aus niedermolekularem Polyethylenglycol (PEG) und höhere molekularem PEG suspendiert bzw. gelöst.

Nach suspendieren bzw. lösen des Wirkstoffs in der Schmelze wird die Zubereitung bis zum Erkalten auf Raumtemperatur gerührt.

Niedermolekulares PEG ist bei Raumtemperatur (etwa 21°C) flüssig, höhermolekulares PEG ist bei Raumtemperatur fest (schneidbar, wachsartig).

Die Mischungsverhältnisse der beiden PEG Typen richten sich nach erforderlicher Viskosität der Zubereitung und nach den mittleren Molekulargewichten der eingesetzten PEG Typen.

Als Beispiele können Zubereitungen aus 6.5 Gewichtsteilen PEG 400 und 1 Gewichtsteil PEG 4000 oder 7 Gewichtsteilen PEG 400 und 2 Gewichtsteilen PEG 6000 verwendet werden. Mischungsbereiche bewegen sich von 1:1 bis 10:1 (jeweils gewichtsteile niedermolekulares PEG zu höhermolekularem PEG). Bevorzug ist der Bereich von 2:1 bis 8:1, besonders bevorzug der Bereich von 3:1 bis 7:1.

Als weitere Gelzubereitung wird ein Polyacrylatgel (sogenanntes "Carbogel"), welches aus 1 Gewichtsteil Carbopol 974 P NF (Hersteller Fa. BF Goodrich, USA) 5 Gewichtsteilen Isopropylalkohol, 5 Gewichtsteil Natronlauge 5 Gew.-% (m/m) und 89 Gewichtsteilen Wasser besteht, eingesetzt. Zur Herstellung der Zubereitung wird das Carbopol mit dem Isopropanol angerieben, der Wirkstoff zugesetzt und die Mischung in Wasser dispergiert. Die Natronlauge wird schrittweise unter Rühren zugegeben. Es bildet sich ein Gel.

### In vivo-Wirkung

### Ocular-Herpes Modell

6 Wochen alte weibliche Mäuse, Stamm BALB/cABom (Gewicht 19 g), werden von einem kommerziellen Züchter (M&B A/S, Dänemark) bezogen und eine Woche später mit HSV infiziert. Die Tiere werden in einem dichten Glasgefäß mit Diethylether (Merck) anästhesiert. Für die oculare Infektion wird die Cornea des rechten Auges mit einer sterilen Kanüle (0,4 x 20mm) dreimal horizontal und dreimal vertikal leicht angeritzt. Die so vorbehandelte Cornea wird mit 5µl Virensuspension (5 x 10⁷ pfu HSV-2G, 7,5 x 10⁷ pfu HSV-1walki) inokuliert. Die infizierten Tiere werden täglich kontrolliert und auf Zeichen einer HSV-Infektion hin untersucht (Blepharitis, Keratitis, Encephalitis). Für betroffene Tiere wird der Krankheitsindex 1 angesetzt, bei symptomfreien Tieren der Krankheitsindex 0. Der jeweilige Krankheitsindex der Gruppe am jeweiligen Tag wird durch Summation über alle Tiere der Gruppe errechnet. Der kumulative Krankheitsindex berechnet sich durch Aufsummieren der Krankheitsindizes einzelner Tage. Die Infektion führt bei 90-100 % der unbehandelten Tiere durch eine generalisierte Infektion mit prominenten zentralnervösen Symptomen im Mittel zwischen 5 und 8 Tagen zum Tode. Moribunde Tiere werden euthanasiert. Die Behandlung erfolgt beginnend 3h nach der Infektion 5x täglich über 5 Tage hinweg durch Aufträufeln von 5µl einer 2 Gew.-% Wirkstoffsuspension in Liquifilm^{©} Augentropfen (Allergan, Ettlingen, Deutschland) (1 ml enthaltend Polyvinylalkohol 14 mg, sowie als Hilfsstoff Chlorobutanol ½ H₂O 5,25 mg , Natriumchlorid) auf das infizierte rechte Auge des Tieres.

### Zosteriform Spread Modell

6 Wochen alte weibliche haarlose Mäuse (C3H/TifBom-hr) werden von einem kommerziellen Züchter (M&B A/S, Dänemark) bezogen und eine Woche später mit HSV infiziert. Die Tiere werden in einem dichten Glasgefäß mit Diethylether (Merck) anästhesiert. Für die dermale Infektion wird die Haut an der rechten Flanke oder im Nackenbereich des Tieres 10x überkreuz mit einer sterilen Kanüle (0,6 x 25mm) angeritzt. Die so vorbehandelte Haut wird mit 10µl Virensuspension (1 x 10⁶ pfu HSV-2G; 1,5 x 10⁶ pfu HSV-1walki) inokuliert. Die Tiere werden täglich inspiziert und der Krankheitsindex anhand folgender Skala bestimmt: 0: keine Infektionsanzeichen sichtbar, 1: Bläschenbildung, 2: leichte Ausbreitung im Dermatom (Zoster), 3: große Areale betroffen (Zoster), 4: konfluente Zosterbande, 5: Lähmung der Extremitäten, 6: Tod. Der jeweilige Krankheitsindex der Gruppe am jeweiligen Tag wird durch Mittelwertbildung über alle Tiere der Gruppe errechnet. Der kumulative Krankheitsindex berechnet sich durch aufsummieren der Krankheitsindizes einzelner Tage. Die Infektion führt bei 90-100 % der unbehandelten Tiere durch eine generalisierte Infektion mit prominenten zentralnervösen Symptomen im Mittel zwischen 5 und 8 Tagen zum Tode. Moribunde Tiere werden euthanasiert. Die Behandlung erfolgt beginnend 6h nach der Infektion 3x täglich über 5 Tage hinweg durch Einreiben der infizierten Körperregion mit einer wirkstoffhaltigen Formulierung.

### Beispiele

### Beispiel 1:

### Wirkung bei topischer Behandlung im ocularen Herpesmodell (ophthalmologische Anwendung)

Die topische Behandlung (Augentropfen) von ocular mit HSV infizierten Tieren zeigt überraschenderweise eine profunde Wirksamkeit der Thiazolylamide (exemplarisch dargestellt durch N-[5-(Aminosulfonyl)-4-methyl-1,3-thiazol-2-yl]-N-methyl-2-[4-(2-pyridinyl)phenyl]acetamid und N-[5-(aminosulfonyl)-4-methyl-1,3-thiazol-2-yl]-2-(2',5'-difluor-1,1'-biphenyl-4-yl)-N-methylacetamid. Im Vergleich zum gegenwärtigen therapeutischen Standard Zovirax© ist eine deutliche Überlegenheit erkennbar. Dies gilt sowohl für das Auftreten von Krankheitserscheinungen am Auge selbst (Blepharitis, Keratitis) als auch für die Verhinderung der Ausbreitung einer generalisierten Herpesinfektion im infizierten Tier (Encephalitis und Tod). Die Wirksamkeit ist für Infektionen mit HSV-1 und HSV-2 gegeben.

Tabelle 1 zeigt die Anzahl der am Tag 16 nach der Infektion überlebenden Tiere von 10 infizierten bei 5x täglicher topischer ophthalmischer Anwendung (von Tag 0 bis Tag 4 nach der Infektion) von 2 Gew.-% Suspensionen der angegebenen Substanzen.

**Tabelle 1:**

| **Substanz** | **HSV-1** | **HSV-2** |
|---|---|---|
| Placebo | 0 | 0 |
| Aciclovir © | 7 | 1 |
| N-[5-(ammosulfonyl)-4-methyl-1,3-thiazol-2-yl]-N-methyl-2-[4-(2-pyridinyl)phenyl]acetamid | 10 | 9 |
| N-[5-(aminosulfonyl)-4-methyl-1,3-thiazol-2-yl]-2-(2',5'-difluor-1,1'-biphenyl-4-yl)-N-methylacetamid | 10 | 4 |

Tabelle 2 zeigt den kumulativen Krankheitsindex vom Tag 0 bis zum Tag 16 nach der Infektion von 10 Tieren bei 5x täglicher topischer ophthalmischer Anwendung (von Tag 0 bis Tag 4 nach der Infektion) von 2 Gew.-% Suspensionen der angegebenen Substanzen.

**Tabelle 2:**

| **Substanz** | **Krankheitsindex HSV-1 Infektion** | **Krankheitsindex HSV-2 Infektion** |
|---|---|---|
| Placebo | 120 | 132 |
| Aciclovir | 33 | 90 |
| N-[5-(aminosulfonyl)-4-methyl-1,3-thiazol-2-yl]-N-methyl-2-[4-(2-pyridinyl)phenyl]acetamid | 0 | 7 |
| N-[5-(aminosulfonyl)-4-methyl-1,3-thiazol-2-yl]-2-(2',5'-difluor-1,1'-biphenyl-4-yl)-N-methylacetamid | 0 | 42 |

### Beispiel 2:

### Wirkung bei topischer Behandlung im Zosteriform Spread Modell

Die topische Behandlung (2 Gew-% Wirkstoffsuspension in 30 Gew.-% Isopropylmyristat, 70 Gew.-% Ethanol) von percutan an der rechten Flanke mit HSV-2 infizierten Tieren zeigt überraschenderweise eine profunde Wirksamkeit der Thiazolylamide (exemplarisch dargestellt durch
- N-[5-(aminosulfonyl)-4-methyl-1,3-thiazol-2-yl]-2-[1,1'-biphenyl]-4-yl-N-methylacetamid
- N-[5-(aminosülfonyl)-4-methyl-1,3-thiazol-2-yl]-2-(2'-fluor[1,1'-biphenyl]-4-yl)-N-methylacetamid
- N-[5-(aminosulfonyl)-4-methyl-1,3-thiazol-2-yl]-2-(3'-fluor-1,1'-biphenyl-4-yl)-N-methylacetamid
- N-[5-(aminosulfonyl)-4-methyl-1,3-thiazol-2-yl]-N-methyl-2-[4-(2-pyridinyl)phenyl]acetamid

Im Vergleich zum gegenwärtigen therapeutischen Standard Zovirax ist eine deutliche Überlegenheit erkennbar.

Tabelle 3 zeigt den kumulativen Krankheitsindex vom Tag 0 bis zum Tag 21 nach der Infektion von 10 Tieren bei 2x täglicher topischer Anwendung auf der Haut (von Tag 0 bis Tag 4 nach der Infektion) von 2 Gew.-% Suspensionen der angegebenen Substanzen.

**Tabelle 3:**

| **Substanz** | **Krankheitsindex** |
|---|---|
| Placebo | 101,4 |
| Aciclovir | 37,5 |
| N-[5-(Aminosulfonyl)-4-methyl-1,3-thiazol-2-yl]-2-(1,1'-biphenyl-4-yl)-N-methylacetamid | 7,9 |
| N-[5-(Aminosulfonyl)-4-methyl-1,3-thiazol-2-yl]-2-(2'-fluor-1,1'-biphenyl-4-yl)-N-methylacetamid | 0,2 |
| N-[5-(Aminosulfonyl)-4-methyl-1,3-thiazol-2-yl]-2-(3'-fluor-1,1'-biphenyl-4-yl)-N-methylacetamid | 0,3 |
| (N-[5-(Aminosulfonyl)-4-methyl-1,3-thiazol-2-yl]-N-methyl-2-[4-(2-pyridinyl)phenyl]acetamid) | 0,3 ' |

### Beispiel 3:

### Wirkung bei topischer Behandlung mit verschiedenen Formulierungen

Als Zubereitungen werden eingesetzt :
- 3 Gew.-% (m/m) Wirkstoff in Wollwachsalkohol Salbe DAB 9 (Zusammensetzung und Herstellung siehe "Streichfähige Zubereitungen").
- 3 Gew.-% (m/m) Wirkstoff in PEG Isogel (aus 6.5 Gewichtsteil PEG 400 und 1 Gewichtsteil PEG 4000, Herstellung siehe "Streichfähige Zubereitungen").
- 3 Gew.-% (m/m) Wirkstoff in Polyacrylatgel (sogenanntes Carbogel) (Herstellung siehe "Streichfähige Zubereitungen").
- Ein wirkstofffreies PEG Isogel (aus 6.5 Gewichtsteil PEG 400 und 1 Gewichtsteil PEG 4000, Herstellung siehe "Streichfähige Zubereitungen", es wird jedoch kein Wirkstoff eingearbeitet) dient als Placebokontrolle.

Die topische Behandlung (verschiedene Formulierungen mit 3 Gew.-% Wirkstoffgehalt) von percutan in der Nackenregion mit HSV-2G infizierten Tieren (Einzelhaltung) zeigt überraschenderweise eine profunde Wirksamkeit der Thiazolylamide (exemplarisch dargestellt durch N-[5-(aminosulfonyl)-4-methyl-1,3-thiazol-2-yl]-N-methyl-2-[4-(2-pyridinyl)phenyl]acetamid mit allen getesteten Formulierungen. Die N-[5-(aminosulfonyl)-4-methyl-1,3-thiazol-2-yl]-N-methyl-2-[4-(2-pyridinyl)phenyl]acetamid PEG Isogelformulierung sowie die Formulierung mit Wollwachsalkohol ist der Zovirax© Augensalbe (GlaxoWellcome^{®}) überlegen. Tabelle 4 zeigt den kumulativen Krankheitsindex vom Tag 0 bis zum Tag 21 nach der Infektion von 10 Tieren bei 2x täglicher topischer Anwendung auf der Haut (von Tag 0 bis Tag 4 nach der Infektion) von 3 Gew.-% Formulierungen der angegebenen Substanzen.

**Tabelle 4:**

| **Substanz** | **Krankheitsindex** |
|---|---|
| Unbehandelt | 99 |
| PEG-Isogel-Placebo | 101 |
| N-[5-(aminosulfonyl)-4-methyl-1,3-thiazol-2-yl]-N-methyl-2-[4-(2-pyridinyl)phenyl]acetamid ***Wollwachsalkohol*** | 1,1 |
| N-[5-(aminosulfonyl)-4-methyl-1,3-thiazol-2-yl)-N-methyl-2-[4-(2-pyridinyl)phenyl]acetamid ***PEG Isogel*** | 0,4 |
| N-[5-(aminosulfonyl)-4-methyl-1,3-thiazol-2-yl]-N-methyl-2-[4-(2-pyridinyl)phenyl]acetamid ***Carbogel*** | 10 |
| Zovirax © Augensalbe (Aciclovir) | 3,6 |

### Beispiel 4:

### Wirkung bei 2-tägiger topischer Behandlung

Als Zubereitungen werden eingesetzt :
- 2 Gew.-% (m/m) Wirkstoff in PEG Isogel (aus 6.5 Gewichtsteil PEG 400 und 1 Gewichtsteil PEG 4000, Herstellung siehe "Streichfähige Zubereitungen").
- Ein wirkstofffreies PEG Isogel (aus 6.5 Gewichtsteil PEG 400 und 1 Gewichtsteil PEG 4000, Herstellung siehe "Streichfähige Zubereitungen", es wird jedoch kein Wirkstoff eingearbeitet) dient als Placebokontrolle.

Die topische Behandlung (2 Gew.-% Wirkstoffgehalt, PEG-Isogel) von percutan in der Nackenregion mit HSV-2G infizierten Tieren (Einzelhaltung) zeigt bei nur 4-maliger Behandlung (2 Tage 2x täglich) überraschenderweise eine profunde Wirksamkeit der Thiazolylamide (expemplarisch dargestellt durch N-[5-(aminosulfonyl)-4-methyl-1,3-thiazol-2-yl]-N-methyl-2-[4-(2-pyridinyl)phenyl]acetamid. Dieses Experiment veranschaulicht nochmals deutlich die hervorragende topische Wirksamkeit der Substanzen. Eine Infektion, welche ohne Behandlung zum Tod aller Versuchstiere führt wird durch nur 4-malige Behandlung völlig supprimiert. Tabelle 5 zeigt den kumulativen Krankheitsindex vom Tag 0 bis zum Tag 21 nach der Infektion von 10 Tieren bei 2x täglicher topischer Anwendung auf der Haut (von Tag 0 bis Tag 1 nach der Infektion) von 2Gew.-% Formulierungen der angegebenen Substanz.

**Tabelle 5:**

| **Substanz** | **Krankheitsindex** |
|---|---|
| Placebo | 96,9 |
| N-[5-(aminosulfonyl)-4-methyl-1,3-thiazol-2-yl]-N-methyl-2-[4-(2-pyridinyl)phenyl]acetamid | 0,3 |

### Ausgangsverbindungen

### Beispiel I

### 2-Chlor-4-methyl-1,3-thiazol-5-sulfonylchlorid

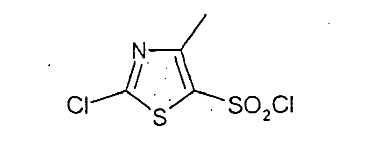

150 g (1,12 mol) 2-Chlor-4-methyl-1,3-thiazol werden bei Raumtemperatur zu einer Lösung von 331 g (2,81 mmol) Thionylchlorid in 653 g (5,61 mmol) Chlorsulfonsäure zugetropft. Die Lösung wird 48 h zum Rückfluss erhitzt. Anschließend wird die Mischung auf 3 1 Eiswasser gegeben und mit 4 x 400 ml Dichlormethan extrahiert. Die vereinigten organischen Phasen werden mit 2,5 l Wasser gewaschen, über Natriumsulfat getrocknet und eingeengt. Nach Destillation des Rohproduktes werden 233,7 g Produkt in Form eines Öls erhalten. (Sdp 87-96°C, 0,7 mbar, GC 98,1%, Ausbeute 89,6%).

### Beispiel II

### 2-Chlor-4-methyl-1,3-thiazol-5-sulfonamid

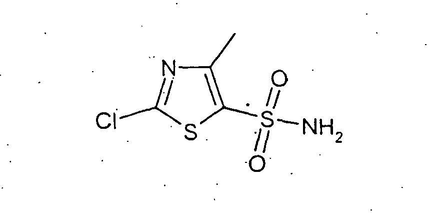

Zu einer Lösung aus 208 g (95 %ig, 0,9 mol) 2-Chlor-4-methyl-1,3-thiazol-5-sulfonylchlorid in 1000 ml Tetrahydrofuran werden 117,7 g (1,8 mol) einer 26 %igen wässrigen Ammoniaklösung bei -10C° zugetropft. Man lässt 2 h ohne weitere Kühlung nachrühren und engt anschließend den Reaktionsansatz am Rotationverdampfer ein. Das Rohprodukt wird ohne weitere Reinigung in die nächste Stufe eingesetzt.

### Beispiel III

### 4-Methyl-2-(methylamino)-1,3-thiazol-5-sulfonamid

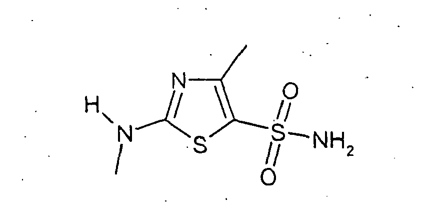

144 g (0,576 mol) 2-Chlor-4-methyl-1,3-thiazol-5-sulfonamid werden bei Raumtemperatur in 600 ml Acetonitril vorgelegt und 147 g (1,9 mol) einer 40 %igen wässrigen Methylamin-Lösung bei Raumtemperatur zudosiert. Der Reaktionsansatz wird 6 h bei 50°C nachgerührt und anschließend am Rotationsverdampfer eingeengt. Der Rückstand wird mit Wasser versetzt, abgesaugt und getrocknet.
Ausbeute: 78 g (66%)
Fp.: 194°C

### Beispiel IV

### 2-Fluorphenylboronsäure

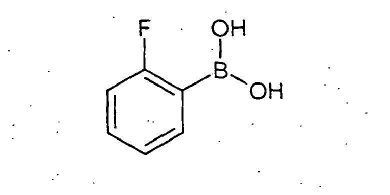

155 g (0,86 mol) 2-Fluorbrombenzol werden unter Argon in 732 ml absolutem Tetrahydrofuran vorgelegt und bei -78°C langsam mit 600 ml 1,6 M n-Butyllithium in Hexan versetzt. Dann wird 2 h bei -78°C nachgerührt. Anschließend werden bei -78°C 298 ml (1,28 mol) Borsäuretrimethylester zugetropft. Nach 1 h wird die Kühlung entfernt und das Reaktionsgemisch über Nacht gerührt und auf Raumtemperatur erwärmt. Zur Aufarbeitung wird der Ansatz mit 346 ml gesättigter Ammoniumchloridlösung bei 0°C versetzt, der pH mit 1N HCl auf 6 eingestellt und die wässrige Phase 3 mal mit je 250 ml Methylenchlorid extrahiert. Die vereinigten organischen Phasen werden mit gesättigter Natriumchloridlösung gewaschen und mit Magnesiumsulfat getrocknet. Man erhält Beispiel IV in Form eines beigen Feststoffs. Ausbeute: 60,0 g (48 %)
MS (EI, m/z): 140 (80%, [M]⁺), 96 (100 %, [C₆H₅F]⁺)

### Beispiel V

### (2'-Fluor[1,1'-biphenyl]-4-yl)essigsäuremethylester

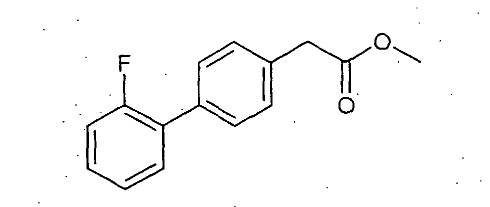

47,6 g (0,21 mol) 4-Bromphenylessigsäuremethylester werden unter Argon in 400 ml absolutem Tetrahydrofuran vorgelegt und bei Raumtemperatur mit 320 ml 1M Sodalösung und 40 g (0,28 mol) 2-Fluorphenylboronsäure versetzt. Nach Zugabe von 7,0 g (0,01 mol) Bis(triphenylphosphan)palladium (II) chlorid wird 18 h unter Rückfluss erhitzt. Nach dem Abkühlen wird mit 500 ml Wasser verdünnt und dreimal mit je 300 ml Essigsäureethylester extrahiert. Die vereinigten organischen Phasen werden mit je 400 ml gesättigter Ammoniumchloridlösung, Wasser und gesättigter Natriumchloridlösung gewaschen, über Magnesiumsulfat getrocknet und am Vakuum vom Solvenz befreit. Man erhält Beispiel V nach Kieselgelfiltration (Petrolether/Essigsäureethylester 10:1) als farbloses Öl. Ausbeute: 46,0 g (94 %)
¹H-NMR (500 MHz, CDCl₃, δ/ppm): 3,71 (s, 2H), 3,76 (s, 3H), 7,18 - 7,46 (m, 4H), 7,40 (d, J = 8,3 Hz; 2H), 7,56 (dd, J₁ = 8,3 Hz, J₂ = 1,7 Hz; 2H).

### Beispiel VI

### (2'-Fluor[1,1'-biphenyl]-4-yl)essigsäure

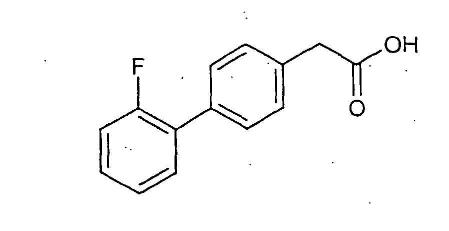

26,5 g (0,11 mol) (2'-Fluoro[1,1'-biphenyl]-4-yl)essigsäuremethylester werden in 50 ml Ethanol vorgelegt und bei Raumtemperatur mit einer Lösung von 12,8 g (0,19 mol) Kaliumhydroxidplätzchen in 25 ml Wasser versetzt. Dann wird 4 h unter Rückfluss erhitzt. Nach dem Abkühlen wird das Rohgemisch am Vakuum eingeengt, der Rückstand in 100 ml Wasser gelöst und mit konz. Salzsäure sauer gestellt. Der Niederschlag wird abfiltriert, mehrfach mit Wasser gewaschen und der Feststoff getrocknet. Man erhält Beispiel VI in Form weißer Kristalle.
Ausbeute: 22,7 g (91 %)
Fp.: 102°C
¹H-NMR (500 MHz, CDCl₃, δ/ppm): 3,74 (s, 2H), 7,18 - 7,47 (m, 4H), 7,41 (d, J = 8,2 Hz; 2H), 7,57 (dd, J₁ = 8,2 Hz, J₂ = 1,6 Hz; 2H).

### Beispiel VII

### [4-(2-Pyridinyl)phenyl]essigsäuremethylester

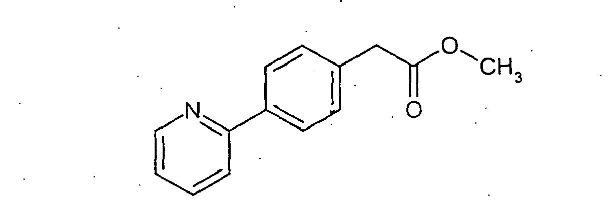

7,85 g (34,3 mmol) 4-Bromphenylessigsäuremethylester werden unter Argon in 95 ml Toluol vorgelegt und bei Raumtemperatur mit 7,97 g (61,7 mmol) Diisopropylethylamin, 9,50 g (37,7 mmol) 2-Trimethylstannylpyridin und 0,4 g (0,3 mmol) Tetrakis(triphenylphosphan)palladium (0) versetzt. Anschließend wird 18 h unter Rückfluss erhitzt. Nach dem Abkühlen wird mit je 100 ml 1N Salzsäure und gesättigter Natriumhydrogencarbonatlösung gewaschen. Die organische Phase wurde verworfen. Die saure und die basische Wasserphase wurden neutral gestellt, mit jeweils 100 ml Dichlormethan extrahiert und die vereinigten organischen Phasen über Magnesiumsulfat getrocknet und am Vakuum vom Solvenz befreit. Man erhält Beispiel VII nach Kieselgelchromatographie (Toluol/Essigsäureethylester Gradient 5:1 - 1:1) als farbloses Öl.
Ausbeute: 1,6 g (19 %)
¹H-NMR (400 MHz, d⁶-DMSO, δ/ppm): 3,64 (s, 3H), 3,76 (s, 2H), 7,33 - 7,40 (m, 1H), 7,39 (d, J = 8,2 Hz; 2 H), 7,86 - 7,90 (m, 1H), 7,96 (d, J = 8,0 Hz; 1H), 8,05 (d, J = 8,2 Hz; 2 H), 8,67 (d, J = 4,2 Hz, breit; 1H).

### Beispiel VIII

### [4-(2-Pyridinyl)phenyl]essigsäure

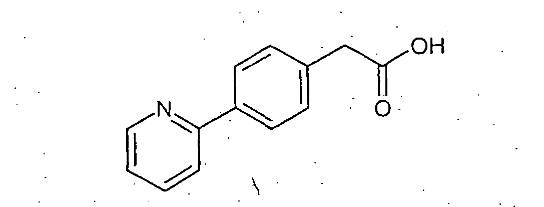

700 mg (3,11 mol) [4-(2-Pyridinyl)phenyl]essigsäuremethylester werden in 5 ml Tetrahydrofuran vorgelegt und bei Raumtemperatur mit 6,2 ml einer 1M Kaliumhydroxidlösung in Wasser versetzt. Dann wird 18 h bei Raumtemperatur gerührt, anschließend das Solvenz am Vakuum weitgehend entfernt, der Rückstand in 10 ml Wasser aufgenommen und mit 2N Salzsäure ein pH-Wert von ca. 5 eingestellt. Zweimalige Extraction der wässrigen Phase mit je 10 ml Dichlormethan lieferte nach Trocknen der vereinigten organischen Phasen über Magnesiumsulfat und Entfernen des Solvenz am Vakuum die Verbindung von Beispiel VIII in Form eines Feststoffs. Ausbeute: 300 mg (46 %)
¹H-NMR (400 MHz, d⁶-DMSO, δ/ppm): 3,76 (s, 2H), 7,45 - 7,51 (m, 1H), 7,50 (d, J = 8,3 Hz; 2H), 8,00 (td, J₁ = 7,7 Hz, J₂ = 1,9Hz; 1H), 8,07 (d, J = 7,9 Hz; 1 H), 8,15 (d, J = 8,3 Hz; 2H), 8,78 (dt, J₁ = 4,0 Hz, J₂ = 0,9Hz; 1H).

### Herstellungsbeispiele

### Beispiel 15

### N-[5-(aminosulfonyl)-4-methyl-1,3-thiazol-2-yl]-2-[1,1'-biphenyl]-4-yl-N-methylacetamid

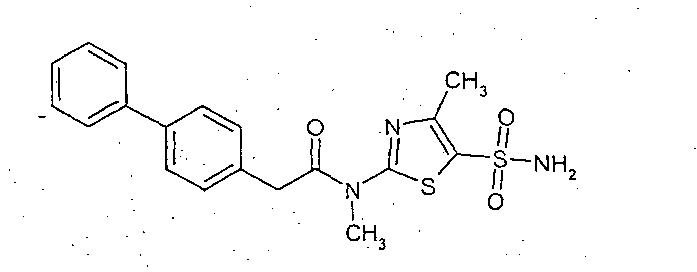

138,2 mg (0,65 mmol) 4-Biphenylessigsäure und 99,7 mg (0,65 mmol) 1-Hydroxy-1H-benzotriazol Hydrat werden in 5 ml Dimethylformamid bei Raumtemperatur vorgelegt. 150 mg (0,72 mmol) 2-Methylamino-4-methyl-1,3-thiazol-5-sulfonamid und 138,7 mg (0,72 mmol) N'-(3-Dimethylaminopropyl)-N-ethylcarbodiimid Hydrochlorid werden zugegeben und der Ansatz 72 h bei Raumtemperatur gerührt. Anschließend wird der Reaktionsansatz abgesaugt und der Rückstand aus 2-Propanol umkristallisiert. Man erhält einen weißen Feststoff.
Ausbeute: 240 mg (83,0 %)
Fp.: 191°C
¹H-NMR (300 MHz, d⁶-DMSO, δ/ppm): 2,47 (s, 3H; teilweise unter DMSO Signal), 3,71 (s, 3H), 4,20 (s, 2H), 7,32 - 7,70 (m, 11H).

### Beispiel 38

### N-[5-(aminosulfonyl)-4-methyl-1,3-thiazol-2-yl]-N-methyl-2-[4-(2-pyridinyl)phenyl]acetamid

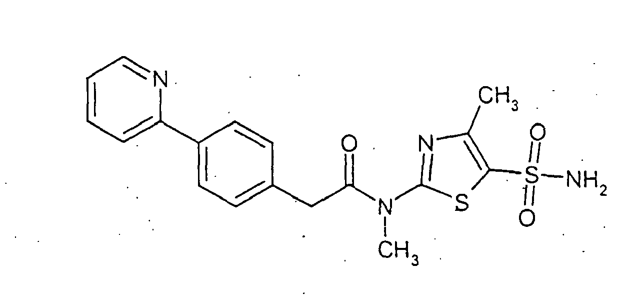

300 mg (1,41 mmol) [4-(2-Pyridinyl)phenyl]essigsäure und 190 mg (1,41 mmol) 1-Hydroxy-1H-benzotriazol Hydrat werden in 4 ml Dimethylformamid bei Raumtemperatur vorgelegt. 307 mg (1,48 mmol) 2-Methylamino-4-methyl-1,3-thiazol-5-sulfonamid und 284 mg (1,48 mmol) N'-(3-Dimethylaminopropyl)-N-ethylcarbodiimid Hydrochlorid werden zugegeben und der Ansatz 18 h bei Raumtemperatur gerührt. Anschließend wird am Vakuum vom Solvenz befreit, der Rückstand in Toluol aufgenommen und das Solvenz erneut am Vakuum entfernt. Der Rückstand wird mit 15 ml Wasser und 3 ml Methanol verrührt, anschließend abfiltriert und das Filtrat mit 20 ml Dichlormethan nachextrahiert. Feststoff und Dichlormethanphase werden vereinigt und das Solvenz am Vakuum entfernt. Man erhält die Verbindung von Beispiel 38 in Form eines weißen Feststoffs.
Ausbeute: 440 mg (74,0 %)
Fp.: 188 - 192°C
MS (ESI, m/z): 403 (100%, [M+H]⁺)
¹H-NMR (400 MHz, d⁶-DMSO, δ/ppm): 2,38 (s, 3H; unter DMSO Signal), 3,64 (s, 3H), 4,15 (s, 2H), 7,28 - 7,26 (m, 1H) ,7,32 (d, J = 8 Hz; 2H), 7,58 (s, 2H), 7,82 7;96 (m, 2H), 7,98 (d, J = 8,0 Hz; 2H), 8,61 (m; 1H).

### Beispiel 57

### N-[5-(aminosulfonyl)-4-methyl-1,3-thiazol-2-yl]-2-(2'-fluor[1,1'-biphenyl]-4-yl)-N-methylacetamid

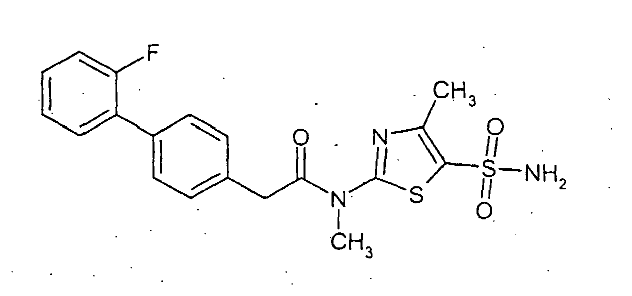

17,33 g (73,3 mmol) (2'-Fluor[1,1'-biphenyl]-4-yl)essigsäure und 9,9 g (73,3 mmol) 1-Hydroxy-1H-benzotriazol Hydrat werden in 600 ml Dimethylformamid bei Raumtemperatur vorgelegt. 16,84 g (81,4 mmol) 2-Methylamino-4-methyl-1,3-thiazol-5-sulfonamid und 15,58 g (81,4 mmol) N'-(3-Dimethylaminopropyl)-N-ethylcarbodiimid Hydrochlorid werden zugegeben und der Ansatz 18 h bei Raumtemperatur gerührt. Das Dimethylformamid wird am Hochvakuum bei 50°C weitgehend entfernt, der Rückstand in 400 ml Dichlormethan aufgenommen und anschließend mit je 350 ml Wasser und 10 % Zitronensäurelösung gewaschen. Man erhält nach Trocknen über Magnesiumsulfat und Entfernen des Solvenz am Vakuum die Verbindung von Beispiel 57 in Form eines weißen Feststoffs.
Ausbeute: 23,2 g (76,0 %)
Fp.: 211°C
¹H-NMR (400 MHz, CDCl₃, δ/ppm): 2,58 (s, 3H), 3,73 (s, 3H), 4,07 (s, 2H), 5,91 (s, 2H), 7,13 - 7,46 (m, 4H), 7,34 (d, J = 8,1 Hz; 2H), 7,56 (d, breit, J = 8,1 Hz; 2H).

### Beispiel 87

### N-[5-(aminosulfonyl)-4-methyl-1,3-thiazol-2-yl]-2-(2',5'-difluor-1,1'-biphenyl-4-yl)-N-methylacetamid

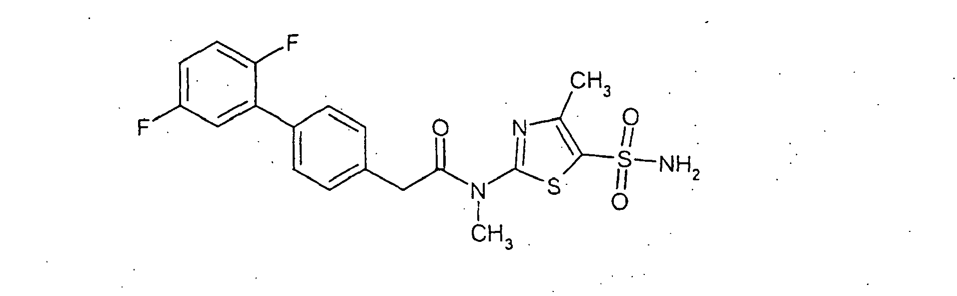

1,00 g (4,0 mmol) (2',5'-Difluor[1,1'-biphenyl]-4-yl)essigsäure und 0,54 g (4,0 mmol) 1-Hydroxy-1H-benzotriazol Hydrat werden in 15 ml Dimethylformamid bei Raumtemperatur vorgelegt. 0,84 g (4,0. mmol) 2-Methylamino-4-methyl-1,3-thiazol-5-sulfonamid und 0,77 g (4,0 mmol) N'-(3-Dimethylaminopropyl)-N-ethylcarbodiimid Hydrochlorid werden zugegeben und der Ansatz 18 h bei Raumtemperatur gerührt. Das Dimethylformamid wird am Hochvakuum bei 50°C weitgehend entfernt, der Rückstand 3 mal mit je 50 ml Wasser ausgerührt, abfiltriert, mit 50 ml Isopropanol verrührt und erneut abfiltriert. Man erhält nach Entfernen des Solvenz am Vakuum die Verbindung von Beispiel 87 in Form eines schwach gelb gefärbten Feststoffs.
Ausbeute: 0,83 g (47,3 %)
Fp.: 184°C
¹H-NMR (400 MHz, DMSO, δ/ppm): 2,49 (s, 3H), 3,71 (s, 3H), 4,24 (s, 2H), 7,22 - 7,46 (m, 3H), 7,38 (d, J = 8,2 Hz; 2H), 7,56 (d, J = 8,2 Hz; 2H), 7,65 (s, 2H).

### Beispiel 126

### N-[5-(Aminosulfonyl)-4-methyl-1,3-thiazol-2-yl]-N-methyl-2-[4-(1H-pyrazol-1-yl)phenyl]acetamid

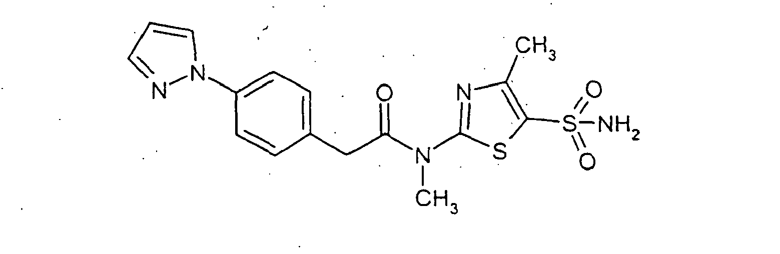

0,100 g (0,48 mmol) 2-Methylamino-4-methyl-1,3-thiazol-5-sulfonamid werden in 10 ml N,N-Dimethylformamid gelöst und bei Raumtemperatur mit 0,110 g (0,53 mmol) [4-(1H-Pyrazol-1-yl)phenyl]essigsäure, 0,070 g (0,53 mmol) 1-Hydroxy-1H-benzotriazol und 0,070 g (0,53 mmol) N,N'-Diisopropylcarbodiimid versetzt. Die Lösung wird über Nacht bei Raumtemperatur gerührt. Anschließend wird die Mischung auf Wasser gegossen und die wässrige Phase dreimal mit Essigsäureethylester extrahiert. Die vereinten organischen Phasen werden mit Natriumsulfat getrocknet und eingeengt. Das Rohprodukt wird an einer präparativen HPLC (RP18-Säule, Laufmittel: Acetonitril-Wasser Gradient) feingereinigt.
Ausbeute: 0,11 g (59 %)
LC-MS (Methode: SMKL-N1-1Low Vol HCl): Retentionszeit: 3,65
MS(ESI): 783 (2Mz+H), 392 (Mz+H)
¹H-NMR (300 MHz, DMSO, δ/ppm): 2,48 (s, 3H), 3,72 (s, 3H), 4,20 (s, 2H), 6,55 (t, J=2Hz; 1H), 7,38 (d, J=7Hz; 2H), 7,65 (s, 2H), 7,75 (d, J=2Hz; 1H), 7,82 (d, J=7Hz; 2H), 8,49 (d, J=2Hz; 1H).

### Beispiel 127

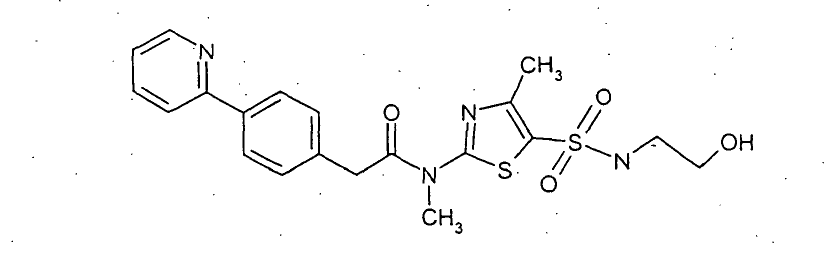

### Methode: SMKL-N1

| | | | | | | |
|---|---|---|---|---|---|---|
| Gerätetyp MS: | Finnigan MAT 900S | | | | | |
| | Ionisierung: | | ESI positiv | | | |
| | | | | | | |
| Gerätetyp HPLC: | TSP : P4000,AS3000,UV3000HR | | | | | |
| Pumpenkopf: | normal | | | | | |
| | | | | | | |
| Säule: | Symmetry C 18 | | | | | |
| | 150 mm x 2,1 mm | | 5 µm | | | |
| Lieferfirma: | Waters | | | | | |
| | | | | | | |
| UV-Detektor DAD: | 210 nm | | | | | |
| Ofentemp.: | 40°C | | | | | |
| Gradient: | Time | A:% | B:% | C:% | D:% | Flow |
| | 0 | 10, 0 | 45 | 45 | -- | 0,6 |
| | 4 | 90 | 5 | 5 | -- | 0,6 |
| | 9 | 90 | 5 | 5 | -- | 0,6 |
| | 9,5 | 10,0 | 45 | 45 | -- | 0,8 |
| | 11,5 | 10,0 | 45 | 45 | -- | 0,8 |
| | 12 | 10,0 | 45 | 45 | -- | 0,6 |
| | | | | | | |
| A: | CH₃CN | | | | | |
| B: | HCl 0,01n | | | | | |
| C: | H₂O | | | | | |
| D: | -- | | | | | |

## Patentansprüche

1. Topisch anwendbare Zubereitungen, enthaltend 0,1 bis 99 Gew.-% einer Verbindung der allgemeinen Formel (I), 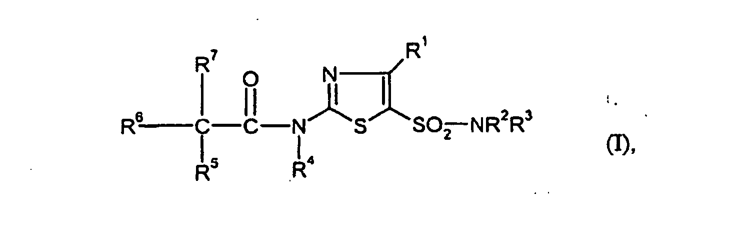 in welcher
R¹ für Wasserstoff, Halogen, (C₁-C₆)-Alkyl, (C₁-C₆)-Alkoxy, Amino-(C₁₋C₆)-alkyl oder Halogen-(C₁-C₆)-alkyl steht,
R² und R³ gleich oder verschieden sind und für Wasserstoff, (C₁-C₆)-Alkoxy, (C₃-C₈)-Cycloalkyl oder Biphenylaminocarbonyl stehen, oder
für (C₁-C₆)-Alkyl stehen, das gegebenenfalls durch 1 bis 3 Substituenten substituiert ist, die aus der Gruppe ausgewählt werden, die aus (C₃-C₆)-Cycloalkyl, (C₁-C₆)-Alkoxy, Halogen, Hydroxy, Amino, Tri-(C₁-C₆)-alkylsilyloxy, Resten der Formel 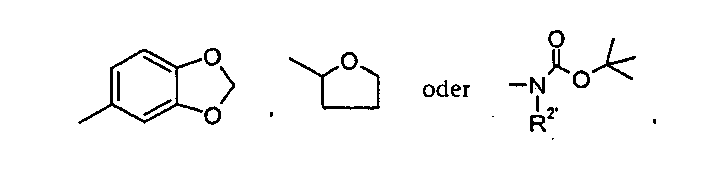 worin R²' für Wasserstoff oder (C₁-C₄)-Alkyl steht,
einem 5- bis 6-gliedrigen aromatischen Heterocyclus mit bis zu 3 Heteroatomen aus der Reihe S, N und/oder O, wobei ein stickstoffhaltiger Heterocyclus auch über das Stickstoffatom gebunden sein kann,
einem gegebenenfalls über ein Stickstoffatom gebundenen 3- bis 8-gliedrigen gesättigten oder ungesättigten, nicht aromatischen, Heterocyclus mit bis zu 3 Heteroatomen aus der Reihe S, N und/oder O, und
(C₆-C₁₀)-Aryl, das seinerseits durch Hydroxy oder (C₁-C₆)-Alkoxy substituiert sein kann, besteht, oder
für eine Gruppe der Formel 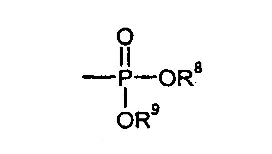 stehen, worin R⁸ und R⁹ gleich oder verschieden voneinander sind und- für Wasserstoff und (C₁-C₄)-Alkyl stehen, oder
für eine Gruppe der Formel 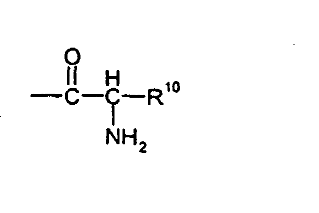 stehen, worin R¹⁰ die Seitengruppe einer natürlich vorkommenden α-Aminosäure darstellt, oder
für eine Gruppe der Formel 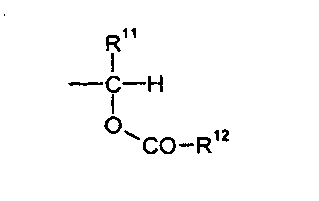 steht, worin R¹¹ für (C₁-C₄)-Alkyl steht, und R¹² für Wasserstoff, (C₁C₄)-Alkyl oder für eine Gruppe der Formel 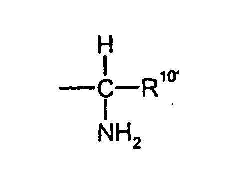 steht, worin R¹⁰ die Seitengruppe einer natürlich vorkommenden α-Aminosäure darstellt, oder
R² und R³ gemeinsam mit dem Stickstoffatom einen 5- bis 6-gliedrigen gesättigten Heterocyclus bildet, der gegebenenfalls noch ein Sauerstoffatom aufweisen kann,
R⁴ für Wasserstoff, (C₁-C₆)-Acyl, (C₂-C₆)-Alkenyl, (C₃-C₈)-Cycloalkyl steht, oder
R⁴ für (C₁-C₆)-Alkyl steht, das gegebenfalls substituiert sein kann durch 1 bis 3 Substituenten, die aus der Gruppe ausgewählt werden, die aus Ha- logen, Hydroxy, (C₃-C₈)-Cycloalkyl, (C₁-C₆)Acyl, (C₁-C₆)-Alkoxy, Carboxyl, 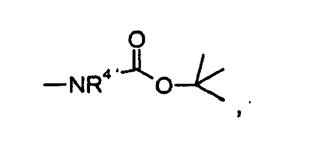 worin R⁴¹ für Wasserstoff steht,
-(OCH₂CH₂)ₙOCH₂CH₃, worin n 0 oder 1 ist, Phenoxy, (C₆-C₁₀)-Aryl und -NR¹³ R¹⁴ besteht,
worin R¹³ und R¹⁴ gleich oder verschieden sind und Wasserstoff, (C₁-C₆)-Acyl, (C₁-C₆)-Alkyl, Carbamoyl, Mono- oder Di(C₁-C₆)-alkylamino(C₁-C₆)alkyl, Mono- oder Di(C₁-C₆)-alkylaminocarbonyl, (C₆-C₁₀)-Aryl oder (C₁-C₆)-Alkoxycarbonyl bedeuten, oder
R¹³ und R¹⁴ gemeinsam mit dem Stickstoffatom einen 5- bis 6 gliedrigen gesättigten Heterocyclus bilden, der gegebenenfalls ein weiteres Heteroatom aus der Reihe S oder O oder einen Rest der Formel -NR¹⁵ enthalten kann, und durch Oxo substituiert sein kann,
worin
R¹⁵ Wasserstoff oder (C₁-C₄)-Alkyl bedeutet, oder
R⁴ für (C₁-C₆)-Alkyl steht, das durch einen 5- bis 6-gliedrigen aromatischen, gegebenenfalls benzokondensierten Heterocyclus mit bis zu 3 Heteroatomen aus der Reihe S, N und/oder O substituiert ist, wobei ein stickstoffhaltiger Heterocyclus auch über das Stickstoffatom gebunden sein kann, oder durch
Reste der Formeln 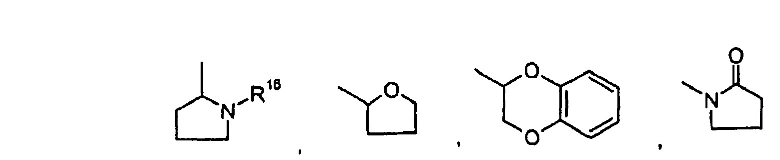 oder 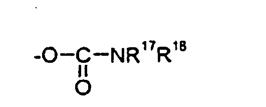 substituiert ist, worin
R¹⁶ Wasserstoff oder (C₁-C₆)-Alkyl bedeutet,
R¹⁷ und
R¹⁸ gleich oder verschieden sind und Wasserstoff, (C₁-C₆) Alkyl oder (C₆-C₁₀)-Aryl bedeuten, wobei zuvor genanntes (C₁-C₆)-Alkyl und (C₆-C₁₀)-Aryl gegebenenfalls durch 1 bis 3 Substituenten substituiert sein können, die aus der Gruppe ausgewählt werden, die aus Hydroxy, (C₁-C₆)-Alkoxy und Halogen besteht,
R⁵ für Wasserstoff, (C₁-C₆)-Alkyl, Halogen, Amino, Mono- oder Di(C₁-C₆)-Alkylamino oder für (C₁-C₆)-Alkanoylamino steht,
R⁶ für Phenyl steht, das gegebenenfalls mit ein bis drei Substituenten substituiert sein kann, die aus der Gruppe ausgewählt werden, die aus
- Halogen,
- (C₆-C₁₀)-Aryl, das gegebenenfalls durch 1 bis 3 Substituenten, ausgewählt aus (C₁-C₆)-Alkanoyl, (C₁-C₆)-Alkoxy, (C₁-C₆)-Alkyl, Halogen, (C₁-C₆)-Alkoxycarbonyl, Nitro, Halogen-(C₁₋C₆)-lkyl, Halogen-(C₁-C₆)-Ikoxy; Amino, (C₁-C₆)-Alkylthio, Hydroxy, Carboxyl, Carbamoyl, Mono- oder Di-(C₁-C₆)-alkylaminocarbonyl, Mono- oder Di-(C₁-C₆)-alkanoylamino, (C₁C₆)-Alkoxycarbonylamino, (C₁-C₆)-Alkylsulfoxy, (C₁-C₆)-Alkylsulfonyl, Tri-(C₁-C₆)-alkylsilyloxy, einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
- (C₁-C₆)-Alkoxy,
- (C₁-C₆)-Alkoxycarbonyl,
- (C₁-C₆)-Alkylthio,
- Hydroxy,
- Carboxyl,
- partiell fluoriertem (C₁-C₆)-Alkoxy mit bis zu 6 Fluoratomen,
- (C₁-C₆)-Alkyl, das gegebenenfalls durch einen Rest der Formel 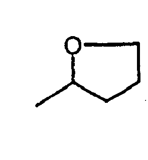 substituiert ist,
- einem gegebenenfalls über ein Stickstoffatom gebundenen 5-bis 6-gliedrigen aromatischen Heterocyclus mit bis zu 3 Heteroatomen aus der Reihe S, N und/oder O, der gegebenenfalls durch 1 bis 3 Substituenten, ausgewählt aus (C₁-C₆)-Alkanoyl, (C₁-C₆)-Alkoxy, (C₁-C₆)-Alkyl, Halogen, (C₁-C₆)-Alkoxycarbonyl, Nitro, Halogen-(C₁-C₆)-alkyl, Halogen-(C₁₋C₆)-alkoxy, Amino, (C₁-C₆)-Alkylthio, Hydroxy, Carboxyl, Carbamoyl, Aminocarbonyl, Mono- oder Di-(C₁-C₆)-alkylaminocarbonyl, Mono- oder Di-(C₁-C₆)-alkanoylamino, (C₁-C₆)-Alkoxycarbonylamino, (C₁-C₆)-Alkylsulfoxy, (C₁-C₆)-Alkylsulfonyl, einem gegebenenfalls über ein Stickstoffatom gebundenen 3- bis 8-gliedrigen gesättigten oder ungesättigten, nicht aromatischen, mono- oder bicyclischen Heterocyclus mit bis zu 3 Heteroatomen aus der Reihe S, N und/oder O, und/oder Cyano substituiert sein kann,
- einem gegebenenfalls über ein Stickstoffatom gebundenen 3-bis 8-gliedrigen gesättigten oder ungesättigten, nicht aromatischen, mono- oder bicyclischen Heterocyclus mit bis zu 3 Heteroatomen aus der Reihe S, N und/oder O, der gegebenenfalls durch 1 bis 3 Substituenten ausgewählt aus Oxo, Halogen, Hydroxy, (C₁-C₆)-Alkoxycarbonyl, (C₁-C₆)-Alkoxycarbonylamino, (C₁-C₆)-Alkyl, Halogen-(C₁-C₆)-alkyl und Hydroxy-(C₁-C₆)-alkyl substituiert sein kann,
- (C₂-C₆)-Alkenyl
und Gruppen der Formeln
- OR¹⁹,
- -NR²⁰R²¹ oder -CO-NR²²R²³,
- Carbazol, Dibenzofuran oder Dibenzothiophen,
- Xanthen oder 9,10-Dihydroacridin besteht,
- worin R¹⁹ Phenyl bedeutet, das seinerseits gegebenenfalls durch eine Gruppe der Formel NR²⁴, R²⁵ substituiert ist,
worin
R²⁴ und R²⁵ gleich oder verschieden sind und Wasserstoff, (C₁-C₆)-Alkyl oder (C₁-C₆)-Acyl bedeuten,
oder
R¹⁹ (C₁-C₆)-Alkyl bedeutet, das gegebenenfalls ein- bis dreifach durch Hydroxy und/oder Halogen substituiert ist,
R²⁰ und R²¹ gleich oder verschieden sind und Wasserstoff, Carbamoyl, Mono- oder Di-(C₁-C₆)-alkylamino carbonyl, Phenyl, (C₁-C₆)-Acyl oder (C₁-C₆)-Alkyl bedeuten,
wobei zuvor genanntes (C₁-C₆)-Alkyl gegebenenfalls durch (C₁-C₆)-Alkoxy, (C₁-C₆)-Acyl, durch Phenyl oder durch einen 5- bis 6-gliedrigen aromatischen Heterocyclus mit bis zu 3 Heteroatomen aus der Reihe S, N und/oder O substituiert ist,
wobei zuvor genanntes Phenyl und zuvor genannter aromatischer Heterocyclus gegebenenfalls ein- bis dreifach gleich oder verschieden durch Halogen und/oder Hydroxy substituiert sind, und
R²² und R²³ gleich oder verschieden sind und Wasserstoff oder (C₁-C₆)-Alkyl bedeuten,
und R⁷ die Bedeutung von R⁵ aufweisen kann und mit dieser gleich oder verschieden sein kann,
und deren Salze,
wobei die Zubereitung ferner zumindest einen weiteren Inhaltsstoff aufweist, der ausgewählt ist aus der Gruppe bestehend aus Augentropfen enthaltend 14 mg Polyvinylalkohol und 5,25 mg Chlorobutanol 1/2 H₂O pro ml, sowie Natriumchlorid, einer Mischung aus 30 Gew.-% Isopropylmyristat und 70 Gew.-% Ethanol, einer Wollwachsalkoholsalbe bestehend aus 0,5 Gewichtsteilen Cetylstearylalkohol, 6 Gewichtsteillen Wollwachsalkohol und 93,5 Gewichtsteilen weißem Vaselin und einem Polyacrylatgel, wobei im Fall der Augentropfen sowie der Mischung aus Isopropylmyristat und Ethanol 2 Gew.-% der Verbindung enthalten sind.

2. Zubereitungen nach Anspruch 1, enthaltend 0,1 bis 99 Gew.-% einer Verbindung der allgemeinen Formel (I).

3. Zubereitungen nach Anspruch 1, enthaltend 0,5 bis 20 Gew.-% einer Verbindung der allgemeinen Formel (I).

4. Zubereitungen nach Anspruch 1, enthaltend 1 bis 5 Gew.-% einer Verbindung der allgemeinen Formel (I)

5. Zubereitungen nach Anspruch 1, enthaltend 2 bis 3 Gew.-% einer Verbindung der allgemeinen Formel (I).

6. Zubereitungen nach Anspruch 1, enthaltend eine Verbindung der allgemeinen Formel (I) in Form einer Suspension oder Salbe.

7. Zubereitungen nach Anspruch 1, enthaltend eine Verbindung der allgemeinen Formel (I) in Form von Lösungen, Sprays, Lotionen, Gelen, Cremes, Pasten, Emulsionen oder Stiften.

8. Zubereitungen nach Anspruch 1 zur Bekämpfung von Erkrankungen.

9. Verwendung von Zubereitungen nach Anspruch 1 zur Herstellung eines Arzneimittels zur Behandlung von viralen Erkrankungen.

## Claims

1. Topically applicable preparations comprising from 0.1 to 95% by weight of a compound of general formula (I) 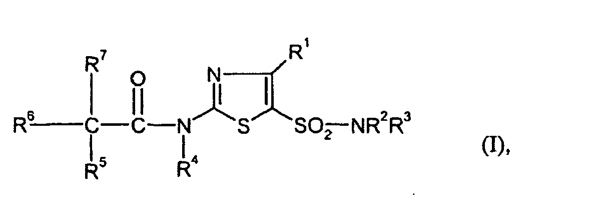 in which
R¹ represents hydrogen, halogen, (C₁-C₆)-alkyl, (C₁-C₆)-alkoxy, amino-(C₁-C₆)-alkyl or halo-(C₁-C₆)-alkyl,
R² and R³ are identical or different and represent hydrogen, (C₁-C₆)-alkoxy, (C₃-C₈)-cydoalkyl or biphenylaminocarbonyl, or
represent (C₁-C₆)-alkyl which is optionally substituted with 1 to 3 substituents which are selected from the group consisting of (C₃-C₆)-cycloalkyl, (C₁-C₆)-alkoxy, halogen, hydroxy, amino, tri-(C₁-C₆)-alkylsilyloxy, radicals of formula 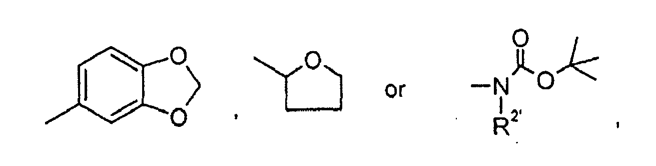 in which R^{2'} represents hydrogen or (C₁-C₄)-alkyl,
a 5- to 6-membered aromatic heterocycle having up to 3 heteroatoms from the series S, N and/or O, whereby a nitrogen-containing heterocycle may also be bonded via the nitrogen atom,
a 3- to 8-membered saturated or unsaturated, nonaromatic, heterocycle which is optionally bonded via a nitrogen atom and has up to 3 heteroatoms from the series S, N and/or O, and
(C₆-C₁₀)-aryl which may in turn be substituted with hydroxy or (C₁-C₆)-alkoxy, or
represent a group of formula 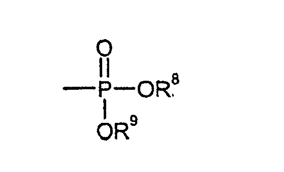 in which R⁸ and R⁹ are identical to or different from one another and represent hydrogen and (C₁-C₄)-alkyl, or
represent a group of formula 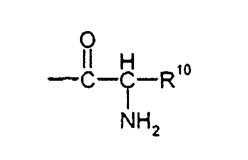 in which R¹⁰ represents the side group of a naturally occurring α-amino acid, or
represents a group of formula 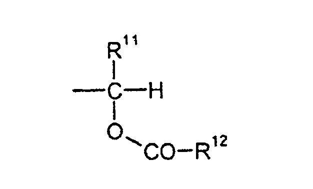 in which R¹¹ represents (C₁-C₄)-alkyl, and R¹² represents hydrogen, (C₁-C₄)-alkyl or a group of formula 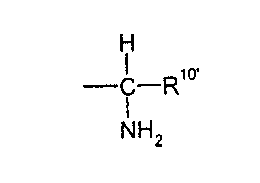 in which R^{10'} represents the side group of a naturally occurring α-amino acid, or
R² and R³ together with the nitrogen atom form a 5- to 6-membered saturated heterocycle which may optionally also comprise an oxygen atom,
R⁴ represents hydrogen, (C₁-C₆)-acyl, (C₂-C₆)-alkenyl, (C₃-C₈)-cycloalkyl, or
R⁴ represents (C₁-C₆)-alkyl which may be optionally substituted with 1 to 3 substituents which are selected from the group consisting of halogen, hydroxy, (C₃-C₈)-cycloalkyl, (C₁-C₆)-acyl, (C₁-C₆)-alkoxy, carboxy, 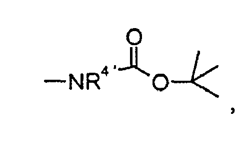 in which R^{4'} represents hydrogen, -(OCH₂CH₂)ₙOCH₂CH₃ in which n is 0 or 1, phenoxy, (C₆-C₁₀)-aryl and -NR₁₃R₁₄,
in which R¹³ and R¹⁴ are identical or different and represent hydrogen, (C₁-C₆)-acyl, (C₁-C₆)-alkyl, carbamoyl, mono- or di(C₁-C₆)-alkylamino(C₁-C₆)-alkyl, mono- or di(C₁-C₆)-alkylaminocarbonyl, (C₆-C₁₀)-aryl or (C₁-C₆)-alkoxycarbonyl or
R¹³ and R¹⁴ together with the nitrogen atom form a 5- to 6-membered saturated heterocycle which may optionally comprise a further heteroatom from the series S or O or a radical of the formula -NR¹⁵, and may be substituted with oxo, in which
R¹⁵ represents hydrogen or (C₁-C₄)-alkyl, or
R⁴ represents (C₁-C₆)-alkyl which is substituted with a 5- to 6-membered aromatic, optionally benzo-fused heterocycle having up to 3 heteroatoms from the series S, N and/or O, whereby a nitrogen-containing heterocycle may also be bonded via the nitrogen atom, or is substituted with radicals of formulae 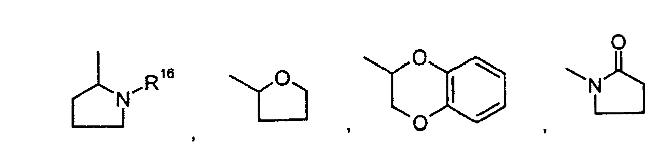 or 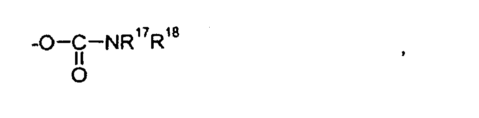 in which
R¹⁶ represents hydrogen or (C₁-C₆)-alkyl,
R¹⁷ and R¹⁸ are identical or different and represent hydrogen, (C₁-C₆)-alkyl or (C₆-C₁₀)-aryl, whereby aforementioned (C₁-C₆)-alkyl and (C₆-C₁₀)-aryl may optionally be substituted with 1 to 3 substituents selected from the group consisting of hydroxy, (C₁-C₆)-alkoxy and halogen,
R⁵ represents hydrogen, (C₁-C₆)-alkyl, halogen, amino, mono- or di(C₁-C₆)-alkylamino or represents (C₁-C₆)-alkanoylamino,
R⁶ represents phenyl which may optionally be substituted with one to three substituents selected from the group consisting of
- halogen,
- (C₆-C₁₀)-aryl which may optionally be substituted with 1 to 3 substituents selected from (C₁-C₆)-alkanoyl, (C₁-C₆)-alkoxy, (C₁-C₆)-alkyl, halogen, (C₁-C₆)-alkoxycarbonyl, nitro, halo-(C₁-C₆)-lkyl, halo-(C₁-C₆)-lkoxy, amino, (C₁-C₆)-alkylthio, hydroxy, carboxy, carbamoyl, mono- or di-(C₁-C₆-) alkylaminocarbonyl, mono- or di-(C₁-C₆)-alkanoylamino, (C₁-C₆)-alkoxycarbonylamino, (C₁-C₆)-alkylsulfoxy, (C₁-C₆)-alkylsulfonyl, tri-(C₁-C₆)-alkylsilyloxy, a 3- to 8-membered, saturated or unsaturated, nonaromatic, mono- or bicyclic heterocycle which is optionally bonded via a nitrogen and has up to 3 heteroatoms from the series S, N and/or O, and/or cyano,
- (C₁-C₆)-alkoxy,
- (C₁-C₆)-alkoxycarbonyl,
- (C₁-C₆)-alkylthio,
- hydroxy,
- carboxy,
- partially fluorinated (C₁-C₆)-alkoxy having up to 6 fluorine atoms,
- (C₁-C₆)-alkyl which is optionally substituted with a radical of formula 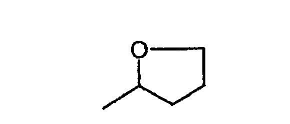
- a 5- to 6-membered aromatic heterocycle which is optionally bonded via a nitrogen atom and has up to 3 heteroatoms from the series S, N and/or O and which may optionally be substituted with 1 to 3 substituents selected from (C₁-C₆)-alkanoyl, (C₁-C₆)-alkoxy, (C₁-C₆)-alkyl, halogen, (C₁-C₆)-alkoxycarbonyl, nitro, halo-(C₁-C₆)-alkyl, halo-(C₁-C₆)-alkoxy, amino, (C₁-C₆)-alkylthio, hydroxy, carboxy, carbamoyl, aminocarbonyl, mono- or di-(C₁-C₆)-alkylaminocarbonyl, mono- or di-(C₁-C₆)-alkanoylamino, (C₁-C₆)-alkoxycarbonylamino, (C₁-C₆)-alkylsulfoxy, (C₁-C₆)-alkylsulfonyl, a 3- to 8-membered saturated or unsaturated, nonaromatic, mono- or bicyclic heterocycle which is optionally bonded via a nitrogen atom and has up to 3 heteroatoms from the series S, N and/or O, and/or cyano,
- a 3- to 8-membered saturated or unsaturated, nonaromatic, mono- or bicyclic heterocycle which is optionally bonded via a nitrogen atom and has up to 3 heteroatoms from the series S, N and/or O, and which may optionally be substituted with 1 to 3 substituents selected from oxo, halogen, hydroxy, (C₁-C₆)-alkoxycarbonyl, (C₁-C₆)-alkoxycarbonylamino, (C₁-C₆)-alkyl, halo-(C₁-C₆)-alkyl and hydroxy-(C₁-C₆)-alkyl,
- (C₂-C₆)-alkenyl
and groups of the formulae
- -OR¹⁹,
- -NR²⁰R²¹ or -CO-NR²²R²³,
- carbazole, dibenzofuran or dibenzothiophene,
- xanthene or 9,10-dihydroacridine,
in which R¹⁹ represents phenyl which in turn is optionally substituted with a group of formula -NR²¹, R²⁵ in which
R²⁴ and R²⁵ are identical or different and represent hydrogen, (C₁-C₆)-alkyl or (C₁-C₆)-acyl, or
R¹⁹ represents (C₁-C₆)-alkyl which is optionally substituted once to three times with hydroxy and/or halogen,
R²⁰ and R²¹ are identical or different and represent hydrogen, carbamoyl, mono- or di-(C₁-C₆)-alkylaminocarbonyl, phenyl, (C₁-C₆)-acyl or (C₁-C₆)-alkyl,
whereby aforementioned (C₁-C₆)-alkyl is optionally substituted with (C₁-C₆)-alkoxy, (C₁-C₆)-acyl, with phenyl or with a 5- to 6-membered
aromatic heterocycle having up to 3 heteroatoms from the series S, N and/or O,
whereby aforementioned phenyl and aforementioned aromatic heterocycle are optionally substituted once to three times, identically or differently, with halogen and/or hydroxy, and
R²² and R²³ are identical or different and represent hydrogen or (C₁-C₆)-alkyl,
and R⁷ may have the meaning of R⁵ and may be identical to or different from the latter,
and the salts thereof,
whereby the preparation further comprises at least one further ingredient selected from the group consisting of eyedrops comprising 14 mg polyvinyl alcohol and 5,25 mg chlorobutanol ½ H₂O per ml as well as sodium chloride, a mixture of 30% by weight isopropyl myristate and 70% by weight ethanol, a wool alcohol ointment consisting of 0,5 parts by weight cetyl stearyl alcohol, 6 parts by weight wool wax alcohol and 93,5 parts by weight white vaseline and a polyacrylate gel, whereby in the case of eyedrops and the mixture of isopropyl myristate and ethanol, the preparation contains 2% by weight of the compound.

2. Preparation according to Claim 1, containing 0,1 to 99% by weight of a compound of general formula (I)

3. Preparation according to Claim 1, containing 0,5 to 20% by weight of a compound of general formula (I)

4. Preparation according to Claim 1, containing 1 to 5% by weight of a compound of general formula (I).

5. Preparation according to Claim 1, containing 2 to 3% by weight of a compound of general formula (I).

6. Preparations according to Claim 1, comprising a compound of general formula (I) in the form of a suspension or an ointment.

7. Preparations according to Claim 1, comprising a compound of general formula (I) in the form of solutions, sprays, lotions, gels, creams, pastes, emulsions, or sticks.

8. Preparations according to Claim 1 for controlling diseases.

9. Use of preparations according to Claim 1 for the production of a medicament for the treatment of viral diseases.

## Revendications

1. Composition topique, contenant 0,1 à 99 % en poids d'un composé de la formule générale (I) : 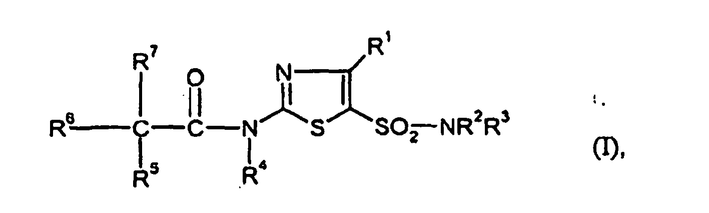 dans laquelle :
R¹ représente hydrogène, halogène, alkyle (C₁-C₆), alcoxy (C₁-C₆), aminoalkyle (C₁-C₆) ou halogénoalkyle (C₁-C₆),
R² et R³ sont identiques ou différents et représentent hydrogène, alcoxy (C₁-C₆), cycloalkyle (C₃-C₈) ou biphénylaminocarbonyle, ou
alkyle (C₁-C₆), qui est le cas échéant substitué par 1 à 3 substituants, qui sont choisis parmi le groupe qui consiste en cycloalkyle (C₃-C₆), alcoxy (C₁-C₆), halogène, hydroxy, amino, tri(alkyl (C₁-C₆))silyloxy, des restes de formule : 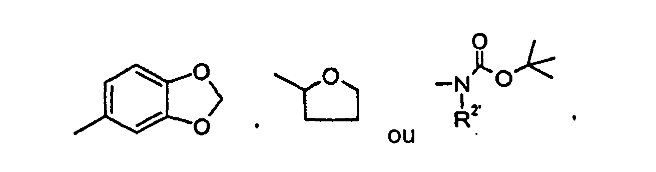
où R^{2'} représente hydrogène ou alkyle (C₁-C₄),
un hétérocycle aromatique ayant 5 à 6 membres avec, jusqu'à 3 hétéroatomes de la série S, N et/ou O, où un hétérocycle azote peut être relié également par un atome d'azote,
un hétérocycle non aromatique, saturé ou insaturé, le cas échéant relié par un atome d'azote, ayant 3 à 8 membres, avec jusqu'à 3 hétéroatomes de la série S, N et/ou O, et
aryle (C₆-C₁₀), qui peut être substitué par hydroxy ou alcoxy (C₁-C₆), ou
représentent un radical de la formule : 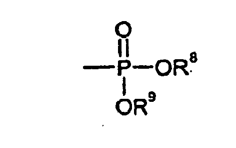 où R⁸ et R⁹ sont identiques ou différents et représentent hydrogène ou alkyle (C₁-C₄), ou
représentent un radical de formule : 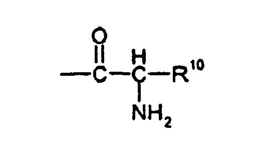 où R¹⁰ représente le groupe latéral d'un acide α-aminé naturel, ou un radical de formule : 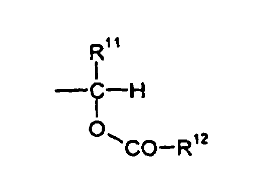 où R¹¹ représente alkyle (C₁-C₄), et R¹² représente hydrogène, alkyle (C₁-C₄) ou un radical de formule : 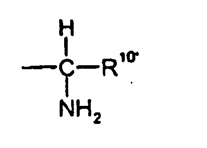 où R¹⁰ représente le groupe latéral d'un acide α-aminé naturel, ou
R² et R³ avec l'atome d'azote, forment un hétérocycle saturé ayant 5 à 6 membres, qui peut présenter le cas échéant, également un atome d'oxygène,
R⁴ représente hydrogène, acyle (C₁-C₆), alcényle (C₂-C₆), cycloalkyle (C₃-C₈), ou
alkyle (C₁-C₆), qui peut être le cas échéant substitué par 1 à 3 substituants, qui sont choisis parmi le groupe, qui consiste en halogène, hydroxy, cycloalkyle (C₃-C₈), acyle (C₁-C₆), alcoxy (C₁-C₆), carboxyle, 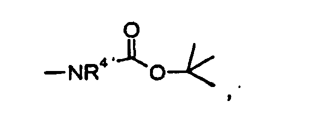 où R^{4'} représente hydrogène,
-(OCH₂CH₂)ₙOCH₂CH₃, où n est 0 ou 1, phénoxy, aryle (C₆-C₁₀) et - NR¹³R¹⁴,
où R¹³ et R¹⁴ sont identiques ou différents, et représentent hydrogène, acyle (C₁-C₆), alkyle (C₁-C₆), carbamoyle, mono- ou di(alkyl (C₁-C₆))aminoalkyle (C₁-C₆), mono- ou di(alkyl (C₁-C₆))aminocarbonyle, aryle (C₆-C₁₀) ou (alcoxy (C₁-C₆))carbonyle, ou
R¹³ et R¹⁴ avec l'atome d'azote, forment un hétérocycle saturé ayant 5 à 6 membres, qui peut contenir le cas échéant, également un autre hétéroatome de la série S ou O ou un reste de formule -NR¹⁵, et qui peut être substitué par oxo,
où R¹⁵ représente hydrogène ou alkyle (C₁-C₄), ou
R⁴ représente alkyle (C₁-C₆), qui est substitué par un hétérocycle aromatique ayant 5 à 6 membres, le cas échéant benzocondensé, ayant jusqu'à 3 hétéroatomes de la série S, N et/ou O, où un hétérocycle azoté peut être lié par l'atome d'azote, ou qui est substitué par un reste de formule : 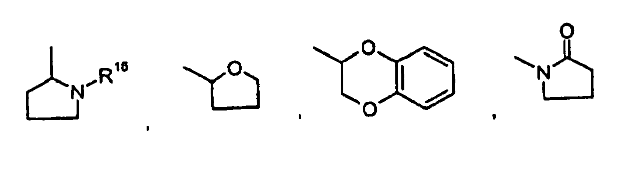 ou 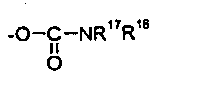 où R¹⁶ représente hydrogène ou alkyle (C₁-C₆),
R¹⁷ et R¹⁸ sont identiques ou différents, et représentent hydrogène, alkyle (C₁-C₆) ou aryle (C₆-C₁₀), où les radicaux alkyle (C₁-C₆) et aryle (C₆-C₁₀) mentionnés peuvent être le cas échéant substitués par 1 à 3 substituants, qui sont choisis parmi le groupe qui consiste en hydroxy, alcoxy (C₁-C₆) et halogène,
R⁵ représente hydrogène, alkyle (C₁-C₆), halogène, amino, mono- ou di(alkyl (C₁-C₆))amino, ou (alkanoyl (C₁-C₆))amino,
R⁶ représente phényle, qui peut être substitué le cas échéant avec un à trois substituants, qui sont choisis parmi le groupe, qui consiste en
- halogène,
- aryle (C₆-C₁₀), qui peut être le cas échéant substitué par 1 à 3 substituants choisis parmi alcanoyle (C₁-C₆), alcoxy (C₁-C₆), alkyle (C₁-C₆), halogène, (alcoxy (C₁-C₆))carbonyle, nitro, halogénoalkyle (C₁-C₆), halogénoalcoxy (C₁-C₆), amino, alkylthio (C₁-C₆), hydroxy, carboxyle, carbamoyle, mono- ou di(alkyl (C₁-C₆))aminocarbonyle, mono- ou di(alcanoyl (C₁-C₆))amino, (alcoxy (C₁-C₆))carbonylamino, (alkyl (C₁-C₆))sulfoxy, (alkyl (C₁-C₆))sulfonyle, tri(alkyl (C₁-C₆))silyloxy, un hétérocycle mono- ou bicyclique, saturé ou insaturé, non aromatique, le cas échéant lié par un atome d'azote, ayant 3 à 8 membres et jusqu'à 3 hétéroatomes de la série S, N et/ou O, et/ou cyano
- alcoxy (C₁-C₆),
- (alcoxy (C₁-C₆))carbonyle,
- alkylthio (C₁-C₆),
- hydroxy,
- carboxyle,
- alcoxy (C₁-C₆) partiellement fluoré avec jusqu'à 6 atomes de fluor,
- alkyle (C₁-C₆), qui est le cas échéant substitué par un reste de formule : 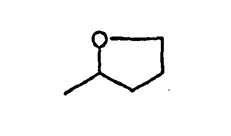
- un hétérocycle aromatique, le cas échéant relié par un atome d'azote, ayant 5 à 6 membres et jusqu'à 3 hétéroatomes de la série S, N et/ou O, qui peut être substitué le cas échéant, par 1 à 3 substituants, choisis parmi alcanoyle (C₁-C₆), alcoxy (C₁-C₆), alkyle (C₁-C₆), halogène, (alcoxy (C₁-C₆))carbonyle, nitro, halogénoalkyle (C₁-C₆), halogénoalcoxy (C₁-C₆), amino, alkylthio (C₁-C₆), hydroxy, carboxyle, carbamoyle, aminocarbonyle, mono- ou di(alkyl (C₁-C₆))aminocarbonyle, mono- ou di(alcanoyl (C₁-C₆))amino, (alcoxy (C₁-C₆))carbonylamino, (alkyl (C₁-C₆))sulfoxy, (alkyl (C₁-C₆))sulfonyle, un hétérocycle mono- ou bicyclique, saturé ou insaturé, non aromatique, le cas échéant lié par un atome d'azote, ayant 3 à 8 membres et jusqu'à 3 hétéroatomes de la série S, N et/ou O, et/ou cyano,
- un hétérocycle mono- ou bicyclique, saturé ou insaturé, non aromatique, le cas échéant relié par un atome d'azote, ayant 3 à 8 membres et jusqu'à 3 hétéroatomes de la série S, N et/ou O, qui peut être substitué le cas échéant, par 1 à 3 substituants, choisis parmi oxo, halogène, hydroxy, (alcoxy (C₁-C₆))carbonyle, (alcoxy (C₁-C₆))carbonylamino, alkyle (C₁-C₆), halogénoalkyle (C₁-C₆) et hydroxyalkyle (C₁-C₆),
- alcényle (C₂-C₆),
et les radicaux de formule
- OR¹⁹,
- NR²⁰R²¹ ou -CO-NR²²R²³,
- carbazole, dibenzofuranne ou dibenzothiophène,
- xanthène ou 9,10-dihydroacridine,
où R¹⁹ représente phényle, qui peut être substitué le cas échéant, par un radical de formule NR²⁴R²⁵,
où
R²⁴ et R²⁵ sont identiques ou différents et représentent hydrogène, alkyle (C₁-C₆) ou acyle (C₁-C₆),
ou
R¹⁹ représente alkyle (C₁-C₆), qui est substitué le cas échéant, une à trois fois, par hydroxy et/ou halogène,
R²⁰ et R²¹ sont identiques ou différents, et représentent hydrogène, carbamoyle, mono- ou di(alkyle (C₁-C₆))aminocarbonyle, phényle, acyle (C₁-C₆) ou alkyle (C₁-C₆),
où le radical alkyle (C₁-C₆) mentionné, est le cas échéant substitué par alcoxy (C₁-C₆), par acyle (C₁-C₆), par phényle ou par un hétérocycle aromatique ayant 5 à 6 membres et jusqu'à 3 hétéroatomes de la série S, N et/ou O,
où le radical phényle mentionné et le radical hétérocycle mentionné, sont le cas échéant substitués une à trois fois, de manière identique ou différente, par halogène et/ou hydroxy, et
R²² et R²³ sont identiques ou différents, et représentent hydrogène alkyle (C₁-C₆),
et R⁷ peut avoir la signification de R⁵ et peut être identique ou différent de celui-ci,
et leurs sels,
où la composition présente en outre, au moins un autre constituant, qui est choisi parmi le groupe consistant en les gouttes oculaires contenant 14 mg de poly(alcool vinylique) et 5,25 mg de chlorobutanol ½ H₂O par ml, ainsi que le chlorure de sodium, un mélange de 30 % en poids de myristate d'isopropyle et de 70 % en poids d'éthanol, une pommade d'alcool de cire de laine consistant en 0,5 partie en poids de cétylstéarylalcool, 6 parties en poids d'alcools de cire de laine et 93,5 parties en poids de vaseline blanche et un gel de polyacrylate, où dans le cas des gouttes oculaires, ainsi que du mélange du myristate d'isopropyle et d'éthanol, 2 % en poids du composé sont présents.

2. Composition selon la revendication 1, contenant 0,1 à 99 % en poids d'un composé de la formule générale (I).

3. Composition selon la revendication 1, contenant 0,5 à 20 % en poids d'un composé de la formule générale (I).

4. Composition selon la revendication 1, contenant 1 à 5 % en poids d'un composé de la formule générale (I).

5. Composition selon la revendication 1, contenant 2 à 3 % en poids d'un composé de la formule générale (I).

6. Composition selon la revendication 1, contenant un composé de la formule générale (I) sous forme d'une suspension ou d'une pommade.

7. Composition selon la revendication 1, contenant un composé de la formule générale (I) sous forme de solutions, sprays, lotions, gels, crèmes, pâtes, émulsions ou crayons.

8. Compositions selon la revendication 1 pour lutter contre les maladies.

9. Utilisation des compositions selon revendication 1 pour la préparation d'un médicament destiné à traiter des maladies virales.
